# EUROPEAN PATENT SPECIFICATION

(11) **EP 1 280 566 B1**
(45) Date of publication and mention of the grant of the patent: **19.08.2009**
(21) Application number: 00928733.5
(22) Date of filing: 01.05.2000
(51) Int. Cl.: A61L 27/54, A61L 27/22, A61L 27/20, A61K 47/48

(54) **GROWTH FACTOR MODIFIED PROTEIN MATRICES FOR TISSUE ENGINEERING**
MATRIZEN AUS MODIFIZIERTEN WACHSTUMFAKTOREN ZUM GEWEBEAUFBAU
MATRICES PROTEIQUES MODIFIEES PAR DES FACTEURS DE CROISSANCE UTILISEES DANS LE DOMAINE DU GENIE TISSULAIRE

(43) Date of publication of application: 05.02.2003
(73) Proprietor: Eidgenossisch Technische Hochschule Zürich, 8092 Zürich (CH); Universität Zürich, 8006 Zürich (CH)
(72) Inventor: HUBBELL, Jeffrey, A., CH-8126 Zumikon (CH); SCHENSE, Jason, C., CH-8008 Zurich (CH); SAKIYAMA-ELBERT, Shelly, E., St.Louis, MO 63130 (US)
(74) Representative: Klunker . Schmitt-Nilson . Hirsch
(86) International application number: PCT/US2000/011947
(87) International publication number: WO 2001/083522

(56) References cited:
- WO-A-00/64481
- DE-U- 20 010 297
- J C SCHENSE & J A HUBBELL: "Cross-linking exogenous bifunctional peptides into fibrin gels with factor XIIIa" BIOCONJUGATE CHEMISTRY, vol. 10, no. 1, January 1999 (1999-01), pages 75-81, XP000867031 WASHINGTON US
- S E SAKIYAMA ET AL.: "Incorporation of heparin-binding peptides into fibrin gels enhances neurite eextension; an example of designer matrices in tissue engineering" FASEB JOURNAL FOR EXPERIMENTAL BIOLOGY, vol. 13, no. 12, December 1999 (1999-12), pages 2214-2224, XP002128749 BETHESDA, MD US

## Description

### Field of the Invention

The invention relates to the use of matrices that contain pharmaceutically active molecules, including fusion proteins of pharmaceutically active molecules, particularly growth factors, using affinity binding interactions, for use in tissue repair or regeneration and/or controlled release of the pharmaceutically active molecules.

### Background of the Invention

For tissue repair or regeneration, cells must migrate into a wound bed, proliferate, express matrix components or form extracellular matrix, and form a final tissue shape. Multiple cell populations must often participate in this morphogenetic response, frequently including vascular and nerve cells. Matrices have been demonstrated to greatly enhance, and in some cases have been found to be essential, for this to occur. Natural cell in-growth matrices are subject to remodeling by cellular influences, all based on proteolysis, e.g. by plasmin (degrading fibrin) and matrix metalloproteinases (degrading collagen, elastin, etc.). Such degradation is highly localized and occurs only upon direct contact with the migrating cell. In addition, the delivery of specific cell signaling proteins such as growth factors is tightly regulated. In the natural model, macroporous cell in-growth matrices are not used, but rather microporous matrices that the cells can degrade, locally and upon demand, as the cells migrate into the matrix.

Controlled delivery devices for growth factors have been designed previously based on use of immobilized heparin to sequester the growth factor of some form. For example, Edelman et al. have used heparin-conjugated SEPHAROSE^{™} beads within alginate. The beads serve as reservoirs that release basic fibroblast growth factor ("bFGF") slowly based on the binding and dissociation of bFGF with heparin.

It has been demonstrated that bi-domain peptides, which contain a factor XIIIa substrate sequence and a bioactive peptide sequence, can be cross-linked into fibrin gels and that this bioactive peptide retains its cellular activity *in vitro* (Schense, J.C., et al. (1999) Bioconj. Chem. 10:75-81). While peptides can partially mimic the bioactivity of the whole protein from which they are derived, this bioactivity is usually lower than the bioactivity of the whole protein, and sometimes it is impossible to mimic certain proteins with only a short peptide. It would therefore be desirable to be able to incorporate the entire protein, such as a growth factor or other pharmaceutically active molecule, into the matrix.
WO98/43686 discloses fusion proteins that contain both a transglutaminase substrate domain such as factor XIII, a substrate domain and a bioactive factor. These peptides are covalently attached to a fibrin substrate having a three-dimensional structure capable of supporting cell growth and the resulting materials are useful in promoting cell growth, wound healing and tissue regeneration.

While delivery systems for proteins and growth factors exist and are known, there remains a need for a matrix for use in tissue repair that promotes cellular migration and tissue in-growth into the matrix through the control of growth factor presentation and release to the migrating cells and control of cellular adhesion sites. The need is particularly great for such in-growth matrices that could locally present growth factors and retain their influence and activity locally, through affinity interactions with the matrix, as occurs in nature.

It is therefore an object of the present invention to provide natural, biodegradable matrices for tissue repair, regeneration, and remodeling having incorporated therein growth factors which retain the activity of the intact growth factor molecules.

It is a further object of the present invention to provide natural, biodegradable matrices for controlled and/or sustained release of growth factors.

### Summary of the Invention

Subject-matter of the present invention is a cross-linked matrix suitable for cellular growth or in-growth as claimed in claim 1, a method for making a matrix as claimed in claim 16, an engineered protein or polysaccharide graft as claimed in claim 25, and the matrix of the present invention for repair, regeneration, or remodelling of tissues and/or release of bioactive factors.

Embodiments of the present invention are claimed in the respective ependent claims.

Proteins are incorporated into protein or polysaccharide polymer matrices or gels for use in tissue repair, regeneration and/or remodeling and/or drug delivery. Generally, the proteins can be incorporated so that they are released by degradation of the matrix, by enzymatic action and/or diffusion. The present invention is directed to release by enzymatic or hydrolytic degradation. As demonstrated by the examples, one method is to bind heparin to the matrix by either covalent or non-covalent methods, to form a heparin-matrix. The heparin then non-covalently binds heparin-binding growth factors to the protein matrix. If the protein to be bound does not contain a native heparin-binding sequence, a fusion protein can be constructed containing the native protein sequence and a synthetic heparin-binding domain. According to the invention, a fusion protein is constructed which contains a crosslinkable region(s) and the native protein sequence, and this fusion protein can be sequestered by cross-linking it to form a matrix. The fusion protein or peptide domain contains degradable linkage that contains hydrolytic or enzymatic cleavage sites. This allows the rate of delivery to be varied at different locations within the matrix depending on cellular activity at that location and/or within the matrix. This approach can be particularly useful when long-term drug delivery is desired, for example in the case of nerve regeneration, where it is desirable to vary the rate of drug release spatially as a function of regeneration, e.g. rapidly near the living tissue interface and more slowly farther into the injury zone. Additional benefits include the lower total drug dose within the delivery system, and spatial regulation of release which permits a greater percentage of the drug to be released at the time of greatest cellular activity. The examples demonstrate optimized ratios or levels of growth_factor, heparin binding domain, and heparin or heparin binding peptide sequestered within the matrix that optimally induced cell in-growth and tissue regeneration.

### Brief Description of the Drawings

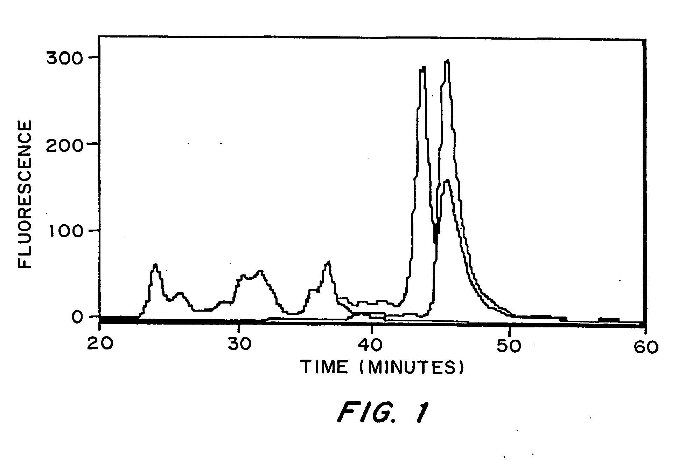

FIGURE 1 is a fluorescence detection chromatograms of plasmin-degraded peptide-containing fibrin gels and free peptide. Size exclusion chromatography of a degraded fibrin gel with the α₂PI₁₋₇-ATIII₁₂₁₋₁₃₄ peptide incorporated, with the same peptide free added to the degraded fibrin gel containing incorporated peptide, and free peptide alone, are shown. The N-terminal leucine residue was dansylated (abbreviated dL). The free peptide eluted at longer times, corresponding to a lower molecular weight, than did the peptide incorporated into the fibrin gel during coagulation, demonstrating covalent attachment to degraded fibrin and thus covalent incorporation via the action of factor XIIIa activity.

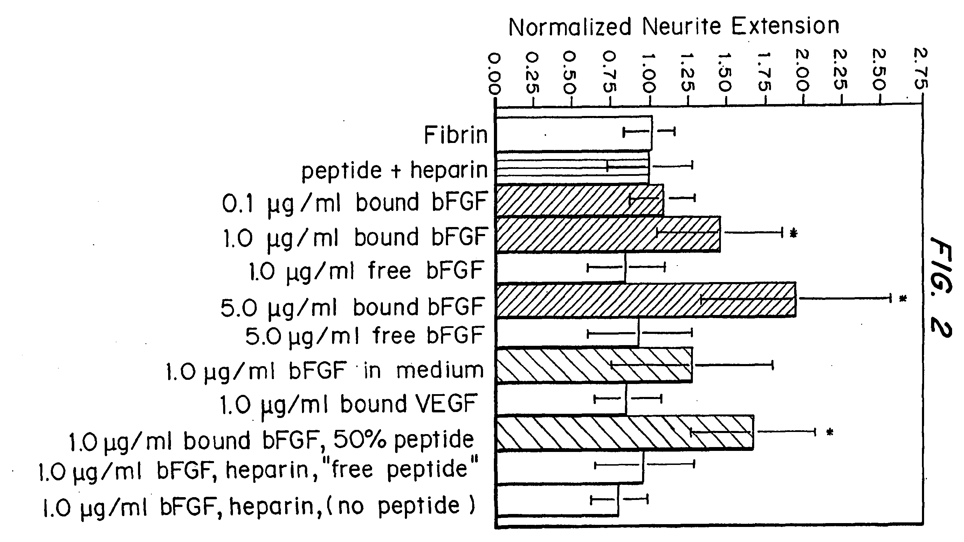

FIGURE 2 is a graph of the effect of matrix bound bFGF on DRG neurite extension at 48 hr. Mean values and standard deviation of the mean are shown. (* denotes p<0.05 compared with unmodified fibrin.)

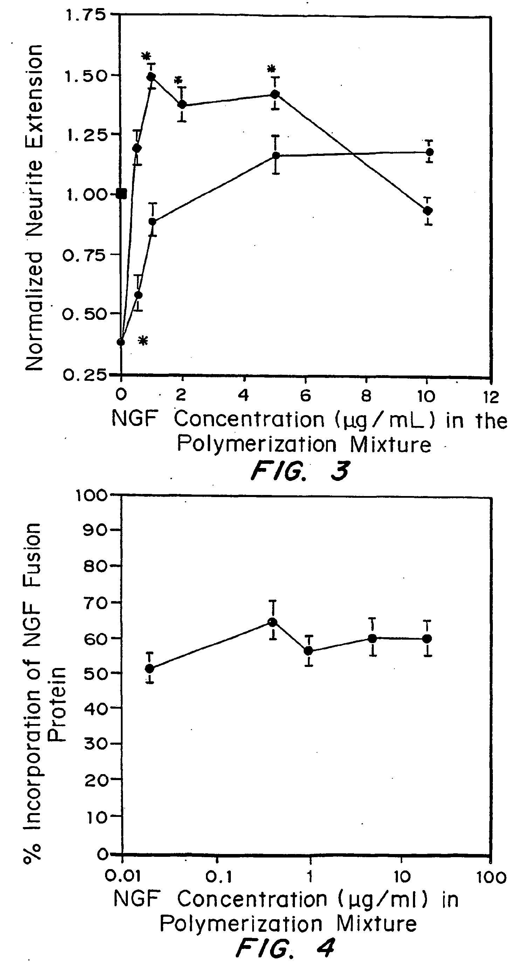

FIGURE 3 is a graph of the effect of immobilized β-NGF fusion proteins on DRG neurite extension within fibrin matrices. Mean values and standard error of the mean are shown. ■ denotes native NGF. ◆ denotes TG-P-NGF. ● denotes TG-Pᵢ-NGF. * denotes p<0.0001 versus unmodified fibrin with NGF in the culture medium. This result demonstrates that matrix-bound β-NGF enhances neurite extension through fibrin matrices versus the same concentration of NGF in the medium.
FIGURE 4 is a graph of the amount of incorporation of β-NGF fusion protein with exogenous factor XIIIa substrate into fibrin matrices, quantified by direct ELISA, using biotin-labeled β-NGF. The incorporation efficiency of the β-NGF fusion protein was relatively constant over the range of concentrations tested.

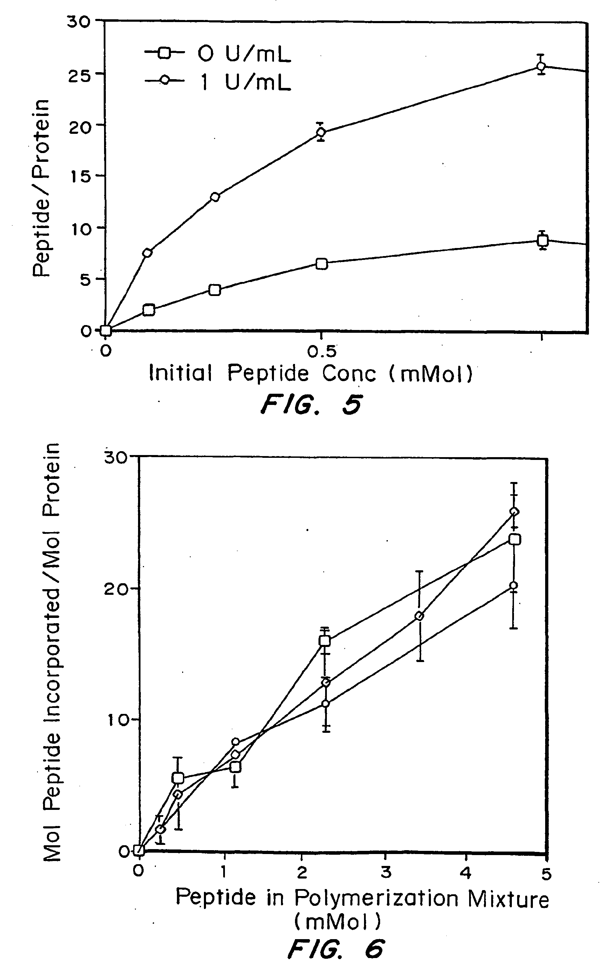

FIGURE 5 is a graph of the incorporation of dLNQEQVSPLRGD (SEQ ID NO:1) into fibrin gels with exogenous Factor XIII added. When 1 U/mL was added, the level of incorporation increased such that more than 25 mol peptide/mol fibrinogen could be achieved.
FIGURE 6 is a graph of the incorporation of the bidomain peptide, dLNQEQVSPLRGD (SEQ ID NO:1) into undiluted fibrin glue. Three separate kits were tested and in each case a high level of incorporation could be observed, reaching 25 mol peptide/mol fibrinogen. The concentration of exogenous peptide required for maximal incorporation was at least 5 mM, possibly due to diffusion limitations within the highly dense fibrin matrix that is created. The level of incorporation was very consistent, with each kit providing a similar incorporation profile.

### Detailed Description of the Invention

As described herein, a method to enhance tissue repair, regeneration or remodeling, using natural matrices having growth factors releasably incorporated therein, has been developed. There are several advantages over the prior art matrices: the natural matrices are biocompatible and biodegradable and can be formed *in vitro* or *in vivo* (in vivo formation is not claimed), at the time of implantation; full length growth factor proteins can be incorporated and retain full bioactivity; the growth factors can be releasably incorporated, using techniques that provide control over how and when and to what degree the growth factors are released, so that the matrix can be used for tissue repair directly or indirectly, using the matrix as a controlled release vehicle.

### I. Matrices and Growth Factors

### A. Matrix Materials

The matrix is formed by crosslinking ionically, covalently, or by combinations thereof, one or more polymeric materials to form a polymeric matrix having sufficient inter-polymer spacing to allow for ingrowth or migration into the matrix of cells.

In the preferred embodiment, the matrix is formed of proteins, most preferably proteins naturally present in the patient into which the matrix is to be implanted. The most preferred protein is fibrin, although other proteins such as collagen and gelatin can also be used. Polysaccharides and glycoproteins may also be used. In some embodiments, it is also possible to use synthetic polymers which are crosslinkable by ionic or covalent binding.

The matrix material is preferably biodegradable by naturally present enzymes. The rate of degradation can be manipulated by the degree of crosslinking and the inclusion of protease inhibitors in the matrix.

### B. Degradable Linkages

The proteins forming the matrix can be modified through inclusion of degradable linkages. Typically, these will be enzyme cleavage sites, such as the site for cleavage by thrombin. Moreover, the fusion proteins or peptide chimeras, which are cross-linked to fibrin gels, are further modified to contain a degradable site between the attachment site (i.e. factor XIIIa substrate or heparin-binding domain) and the bioactive protein (i.e., growth factor or enzyme). These sites may be degradable either by non-specific hydrolysis (i.e. an ester bond) or they may be substrates for specific enzymatic (either proteolytic or polysaccharide degrading) degradation. These degradable sites allow the engineering of more specific release of bioactive factor from fibrin gels. For example, degradation based on enzymatic activity allows for the release of bioactive factors to be controlled by a cellular process rather than by diffusion of the factor through the gel.

The degradation sites allow the bioactive factor to be released with little or no modification to the primary protein sequence, which may result in higher activity of the factor. In addition, it allows the release of the factor to be controlled by cell specific processes, such as localized proteolysis, rather than diffusion from some porous materials. This allows factors to be released at different rates within the same material depending on the location of cells within the material. Cell specific proteolytic activity is vital in applications such as nerve regeneration, which occur over long periods of time. This also reduces the amount of total growth factor needed, since its release is controlled by cellular processes. Conservation of growth factor and its bioavailability are distinct advantages of exploiting cell specific proteolytic activity over the use of diffusion controlled release devices which characteristically result in the loss of a significant amount of bioactive factor in an initial burst release.

Enzymes that could be used for proteolytic degradation are numerous. Proteolytically degradable sites could include substrates for collagenase, plasmin, elastase, stromelysin, or plasminogen activators. Exemplary substrates are listed below. P1-P5 denote amino acids 1-5 positions toward the amino terminus of the protein from the site were proteolysis occurs. P1'-P4' denote amino acids 1-4 positions toward the carboxy terminus of the protein from the site where proteolysis occurs.

**Table 1: Sample substrate sequences for protease.**

| Protease | P5 | P4 | P3 | P2 | P1 | P1' | P2' | P3' | P4' | Reference | SEQ ID NO |
|---|---|---|---|---|---|---|---|---|---|---|---|
| Plasmin | | | L | I | K | M | K | P | | Takagi and Doolittle, (1975) Biochem. 14:5149-5156 | 2 |
| Plasmin | | | N | F | K | S | Q | L | | Takagi and Doolittle, 1975 | 3 |
| Stromelysin | Ac | G | P | L | A | L | T | A | L | Smith et al., (1995). J. Biol. Chem. 270:6440-6449 | 4 |
| Stromelysin | | Ac | P | F | E | L | R | A | NH₂ | Smith et al., 1995 | 5 |
| Elastase | | | Z- | A | A | F | A | NH₂ | | Besson et al., (1996) Analytical Biochemistry 237:216-223. | 6 |
| Collagenase | | G | P | L | G | I | A | G | P | Netzel-Arnett et al., (1991) J. Biol. Chem. 266:6747-6755 | 7 |
| t-PA | P | H | Y | G | R | S | G | G | | Coombs et al., 1998. J. Biol. Chem. 273:4323-4328 | 8 |
| u-PA | P | G | S | G | R | S | A | S | G | Coombs et al., 1998 | 9 |

***Polysaccharide substrates:*** Enzymatic degradation can occur with polysaccharide substrates for enzymes such as heparinase, heparitinase, and chondroitinase ABC. Each of these enzymes have polysaccharide substrates. By virtue of the presence of heparin in all of the heparin-binding systems, the substrate for heparinase is already built into these systems.

***Proteolytic substrates:*** Proteolytic substrate could be added during the peptide synthesis of either the peptide chimera or the heparin-peptide chimera. The heparin-binding peptide chimera could be modified to contain a proteolytic degradation sequence by inserting a protease substrate, such as one of the one for plasmin described above, between the factor XIIIa substrate and the heparin-binding domain. The heparin-peptide chimera could be modified to contain a proteolytic degradation sequence by inserting a protease substrate, such as one of the one for plasmin described above, between the factor XIIIa substrate and the heparin domain. A substrate with a high Kₘ and a low k_{cat} could be used to slow cleavage while occupying active sites of the protease. The cleavage substrates other than those for plasmin could be used to allow release of the bioactive factors to be independent of matrix degradation.

***Oligo-esters:*** An oligo-ester domain could be inserted between the factor XIIIa substrate and either the heparin-binding domain or the heparin domain of the chimera during the peptide synthesis step as well. This could be accomplished using an oligo-ester such as oligomers of lactic acid.

Non-enzymatic degradation substrate can consist of any linkage which undergoes hydrolysis by an acid or base catalyzed mechanism. These substrates can include oligo-esters such as oligomers of lactic or glycolic acid. The rate of degradation of these materials can be controlled through the choice of oligomer.

### C. Heparin; Heparin Binding Peptides

The matrix can be modified through the inclusion of heparin and/or heparin binding fragments, which bind directly or indirectly to proteins which bind to heparin. In the latter case, the peptide can bind to heparin, which is then available for binding to factors which include a heparin binding site, or the peptide can itself contain a heparin portion which is bound by certain heparin-binding growth factors. These can be attached to the matrix material using standard techniques, as discussed in more detail below.

In a preferred embodiment, heparin is attached to fibrin gels non-covalently using a two-part system consisting of a peptide chimera and heparin itself. The peptide chimera consists of two domains, a factor XIIIa substrate and a polysaccharide-binding domain. Once the peptide chimera is cross-linked into the fibrin gel, it attaches the heparin (or other polysaccharides) by non-covalent interactions.

Numerous proteins have been found to have heparin-binding affinity. Some of these proteins and the sequences of their heparin-binding domains are listed below in Table 2. These are also discussed under the section relating to bioactive factors which can be delivered by the polymeric matrix.

**Table 2: Heparin-binding sequences**

| Protein | Heparin-binding domain | Reference | SEQ ID NO |
|---|---|---|---|
| Anti-thrombin III | K( A)FAICLAARLYRKA | ( Tyler-Cross, R, et al, (1994). Protein Science. 3:620-627) | 10 |
| Platelet Factor 4 | YKKIIKKL | Zucker and Katz, (1991). Exper. Biol. Med.:693-702 | 11 |
| Neural Cell Adhesion Molecule | KHKGRDVILKKDVR | Kallapur, et al, (1992) J. Neurosci. Res. 33:538-548 | 12 |
| Fibronectin | YEKPGSPPREVVPRPRPCV KNNQKSEPLIGRKKT | Haugen, et al, (1992). J. Neurosci. 12:2034-2042 | 13 14 |
| bFGF (basic fibroblast growth factor) | KDPKRL YRSRKY | SwissPROT: P09038 | 15 16 |
| aFGF (acidic fibroblast growth factor) | YKKPKL | SwissPROT: P05230 | 17 |
| LPL (lipoprotein lipase) | AKRSSKM CRKRCN | Hata, et al., J. Biol. Chem. 268:8447-8457 | 18 19 |

### D. Bioactive Factors

Many growth factors that are involved in morphogenesis, both in the developing organism and in the adult, bind to extracellular matrix molecules. This affinity provides for a local mode of action of the morphogen, preventing uncontrolled distal influences. The principal matrix affinity interaction that is involved in this localization of influence is for heparin and heparin-sulfate proteoglycans. Growth factors that bind to heparin include the transforming growth factor ("TGF")-beta superfamily (including the bone morphogenic proteins, "BMPs"), the fibroblast growth factor ("FGF") family, and vascular epithelial growth factor ("VEGF"), among others. In general, a growth factors which are considered to bind heparin will elute from a heparin-affinity column at NaCl concentrations above physiological levels (greater than or equal to 140 mM). Additional "heparin-binding" growth factors include interleukin-8, neurotrophin-6, heparin-binding epidermal growth factor, hepatocyte growth factor, connective tissue growth factor, midkine, and heparin-binding growth associated molecule. These growth factors have been shown to regulate tissue repair.

Heparin-binding domains naturally occur in many different families of growth factors. One of these families with one or more member that bind heparin are the fibroblast growth factors (Presta, M., et al., (1992). Biochemical and Biophysical Research Communications. 185:1098-1107). Additional growth factors which bind heparin include transforming growth factor, bone morphogenetic factor, interleukin-8, neurotrophin-6, vascular endothelial cell growth factor, heparin-binding epidermal growth factor, hepatocyte growth factor, connective tissue growth factor, midkine, and heparin-binding growth associated molecule (Götz, R., et al, (1994). Nature. 372:266-269; Kaneda, N., et al, (1996) J. Biochem. 119:1150-1156; Kiguchi, K., et al, (1998) Mol. Carcinogensis. 22:73-83; Kinosaki, M., et al, (1998). Biochim. Biophys. Acta. 1384:93-102; McCaffrey, T., et al, (1992) J. Cell. Physiol. 152:430-440; Nolo, R., et al, (1996) Eur. J. Neurosci. 8:1658-1665; Spillmann, D., et al, (1998). Journal of Biological Chemistry. 273:15487-15493; Steffen, C., et al, (1998); Growth Factors. 15:199-213.

Tessler, S., et al, (1994) J. Biol. Chem. 269:12456-12461). These factors have shown the potential to enhance healing in many different types of tissue including vasculature, skin, nerve and liver. Therefore, these materials can be used to enhance wound healing in many different parts of the body by selecting the appropriate growth factor.

### II. Methods for incorporation and/or Release of bioactive factors.

In the preferred embodiment for incorporation of a growth factor or other bioactive protein within the matrix, the matrix is formed of fibrin and the exogenous molecules incluse a substrate which will be incorporated within fibrin during coagulation. Exogenous peptides according to the invention are designed to include two domains, one domain which is a substrate for a crosslinking enzymes such as XIIIa. Factor XIIIa is a transglutaminase that is active during coagulation. This enzyme, formed naturally from factor XIII by cleavage by thrombin, functions to attach fibrin chains to each other via amide linkages, formed between glutamine side chains and lysine side chains. The enzyme also functions to attach other proteins to fibrin during coagulation, e.g. the protein alpha 2 plasmin inhibitor. The N-terminal domain of this protein, specifically the sequence NQEQVSP (SEQ ID NO:20), has been demonstrated to function as an effective substrate for factor XIIIa. A further domain on this peptide can then be selected to be a bioactive factor, such as a peptide, or a protein, or a polysaccharide (Sakiyama-Elbert, et aL, (2000) J. Controlled Release 65:389-402) and herein. As such, exogenous bioactive factors may be incorporated within fibrin during coagulation via a factor XIIIa substrate.

### A. Use of heparin affinity in release of proteins

A simple way to incorporate many bioactive proteins of interest in healing and regeneration into fibrin is to attach heparin by one of the methods described herein to the fibrin gels and use the heparin to sequester heparin-binding proteins, such as heparin-binding growth factors. This can be accomplished one of two ways, either indirectly by cross-linking a heparin-binding peptide into the fibrin gel and binding heparin to this peptide non-covalently (using a bi-functional peptide containing a heparin-binding domain and a factor XIIIa substrate), or by directly coupling a heparin-peptide chimera (where the heparin is chemically attached to a peptide containing a factor XIIIa substrate). Regardless of the method of incorporation, the incorporated heparin can then sequester proteins, such as growth factors with heparin binding affinity, in the fibrin gel in a manner similar to the way that they are sequestered to the extracellular matrix in nature. Heparin can also protect these factors from proteolytic degradation and prolong their activity until they are released from the matrix.

### Incorporation of heparin through the incorporation of a heparin-binding peptide

The attachment of heparin, either covalently or non-covalently to fibrin gels, adds a novel functionality to these materials. The attachment of heparin permits the fibrin matrix to bind heparin-binding proteins, including growth factors in a manner which does not harm the protein, and prevents free diffusion of the protein from the gel. This allows for the controlled-release of heparin-binding proteins by one of two mechanisms, either degradation of the gel or binding of the protein to some other high affinity protein, such as a cell surface receptor.

Heparin can be attached to fibrin gels non-covalently using a two-part system consisting of a peptide chimera and heparin itself. The peptide chimera consists of two domains, a factor XIIIa substrate and a polysaccharide-binding domain. Once the peptide chimera is cross-linked into the fibrin gel, it attaches the heparin (or other polysaccharides) by non-covalent interactions.

In order to sequester growth factors which do not spontaneously bind heparin, it is necessary to modify the protein through the addition of a functionality capable of attaching to fibrin. This can be accomplished in several ways. By way of example, this may be achieved through the addition of a factor XIIIa substrate or by adding a heparin-binding domain to the resulting fusion protein.

The addition of a synthetic factor XIIIa substrate can be accomplished by expressing a fusion protein containing the native growth factor sequence and a factor XIIIa substrate at either the amino or carboxyl terminus of the fusion protein. This modification is done at the DNA level. Whole proteins present difficulty in that they are synthesized by solid phase chemical synthesis. The DNA sequence encoding the growth factor is adapted to optimal codon usage for bacterial expression. The DNA sequence is then determined for the desired Factor XIIIa substrate, using codons which occur frequently in bacterial DNA.

A series of gene fragments is designed prior to the DNA synthesis. Due to the error frequency of most DNA synthesis, which contains an error approximately every 50 bp, genes are constructed to be approximately 100 bp in length. This reduces the number of colonies that must be screened in order to find one containing the proper DNA sequence. The location at which one gene ends and the next begins is selected based on the natural occurrence of unique restriction enzyme cut sites within the gene, resulting in fragments (or oligonucleotides) of variable length. The process is greatly assisted by the use of software which identifies the location and frequency of restriction enzyme sites within a given DNA sequence.

Once the gene fragments have been successfully designed, common restriction enzyme sites are included on the ends of each fragment to allow ligation of each fragment into a cloning plasmid. For example, adding EcoRI and HindIII sites to each gene fragment allows it to be inserted into the polylinker cloning region of pUC 19. The 3' and 5' single strands of each gene fragment are then synthesized using standard solid phase synthesis with the proper sticky ends for insertion into the cloning vector. Following cleavage and desalting, the single stranded fragments are then purified by PAGE and annealed. After phosphorylation, the annealed fragments are ligated into a cloning vector, such as pUC 19.

Following ligation, the plasmids are transformed into DH5 -F' competent cells and plated on Isopropyl -D-Thiogalactopyranoside(IPTG)/ 5-Bromo-4-chloro-3-indolyl -D-Galactopyranoside (X-gal) plates to screen for insertion of the gene fragments. The resulting colonies which contain gene fragment are then screened for insertion of the proper length. This is accomplished by purifying plasmid from colonies of transformed cells by alkaline lysis miniprep protocol and digesting the plasmid with the restriction enzyme sites present at either end of the gene fragment. Upon detection of the fragments of the proper length by agarose gel electrophoresis, the plasmids are sequenced.

When a plasmid containing a gene fragment with the proper sequence is identified, the fragment is then cut out and used to assemble the full gene. Each time one plasmid is cut with the enzymes at the insertion points and purified from an agarose gel after dephosphorylation of the plasmid. Meanwhile, a second plasmid containing the fragment to be inserted is also cut and the fragment to be inserted is purified from an agarose gel. The insert DNA is then ligated into the dephosphorylated plasmid. This process is continued until the full gene is assembled. The gene is then moved into an expression vector, such as pET 14b and transformed into bacteria for expression. After this final ligation, the full gene is sequenced to confirm that it is correct.

Expression of the fusion protein is accomplished by growing the bacteria until they reach mid-log phase growth and then inducing expression of the fusion protein. Expression is continued for approximately 3 hours and the cells are then harvested. After obtaining a bacterial cell pellet, the cells are lysed. The cell membranes and debris are removed by washing the cell lysate pellet with Triton X100, leaving the inclusion bodies in relatively pure form. The fusion protein is solubilized using high urea concentrations and purified by histidine affinity chromatography. The resulting protein is then renatured gradually by dialysis against a slowly decreasing amount of urea and lyophilized.

### Incorporation of heparin through the incorporation of a heparin-peptide chimera

Polysaccharide grafts (heparin - factor XIIIa substrate peptide chimera) can be incorporated within fibrin during coagulation to provide immobilized heparin sites to bind growth factors and slow their release. Heparin (or other polysaccharides such as heparan sulfate or chondroitin sulfate) can be attached to fibrin directly using factor XIIIa by constructing a heparin-peptide chimera. This chimera contains two domains, a peptide domain consisting of a factor XIIIa substrate and the polysaccharide domain such as heparin. These chimeras are made using modified heparin (or another polysaccharide), e.g. which contains a unique reactive group at one end to control the site where peptide coupling occurs on the heparin molecule. Through the use of a unique functional group on the peptide, such as a side chain present only on the end of the peptide where coupling is desired, the location of coupling on the peptide can be controlled as well. It is also possible to incorporate the factor XIIIa substrate peptide along the chain of heparin, as opposed to at the terminus, and even to incorporate more than one such peptide per heparin chain, but the preferred approach is to incorporate a single factor XIIIa substrate peptide at one end of the heparin. These chimeras can then be covalently cross-linked to fibrin gels during coagulation by the enzymatic activity of factor XIIIa, allowing direct attachment of heparin to the fibrin gel.

### Role of concentration of heparin in determining the release rate of proteins

Determination of the optimal ratios of growth factor:heparin-peptide chimera or the optimal ratio of growth factor:heparin:heparin binding peptide as well as the optimal density of the heparin-peptide chimera or the heparin binding peptide incorporated into the fibrin is important. Despite their relatively strong affinity for heparin, heparin-binding growth factors dissociate from the matrix on a short time scale. Therefore, a high excess of binding sites ensures that the growth factor does not diffuse far before binding to the matrix again. This equilibrium also allows for the binding of free growth factor to cell surface receptors that are in close proximity to the site of dissociation. This method of controlled release provides both relatively long term binding of growth factors and rapid release of growth factors to local cells. As described herein, however, it is not always the case that a high ratio, and thus a slow rate of release, provides the most desirable biological response. There may be cases where more rapid rates of release are desirable, especially with some growth factors.

It has been attempted to mathematically predict what ratio of binding site to growth factor is optimal for cell growth applications as determined by very slow growth factor release. It was demonstrated that the rate of passive release of growth factor from the matrix could be modulated through the ratio of peptide and heparin to the heparin-binding growth factor, higher excesses of heparin and heparin-binding peptide relative to the heparin-binding growth factor leading to slower passive release. It is not always possible to use such methods to predict the optimal release characteristics, however. For example, the examples demonstrate that BMP-2 may be advantageously released more rapidly from a material with a ratio of heparin and heparin-binding peptide to BMP-2 that is closer to equimolar. In some case better results are obtained with lower heparin to growth factor ratios.

The ability of the heparin-containing delivery system to deliver growth factors in an active form was tested in an ectopic model of bone formation, where fibrin matrices containing the delivery system and BMP-2 were implanted subcutaneously in rats. In this model, favorable conditions for bone formation were at low heparin to growth factor ratios of 1:1. The addition of higher ratios, such as 5:1 or greater, were *inhibitory* to bone formation. These results were unexpected based on previous published studies and suggest that low heparin to growth factor ratios have unanticipated use in delivery of BMP-2. (See Example 5).

Addition of exogenous factor XIII to fibrinogen preparations can be used to increase the number of heparin-binding peptides incorporated with fibrin matrices, allowing the heparin-binding peptide to serve both as an immobilization site for heparin and a cell adhesion site. This increase in peptide concentration might enhance the ability of such materials to promote neurite extension, and other forms of cell migration, through the use of heparin-binding domains as cell adhesion sites. It has been demonstrated that heparin-binding peptides act as adhesion peptides and as such can enhance the rate of cell migration within fibrin (Sakiyama, S.E., et al., (1999) FASEB J. 13:2214-2224). However, this response required ca. 8 moles of incorporated peptide per mole of fibrinogen in the clot. As such, use of a large number of these heparin-binding peptides to bind heparin for use as an affinity site in the sustained release of heparin-binding growth factors can remove the beneficial adhesion effect of the heparin binding peptide. This limitation can be overcome by incorporation of higher levels of the exogenous heparin-binding peptide, which can be accomplished through the addition of higher levels of factor XIIIa to the fibrinogen preparation, thus permitting the peptide to have both effects simultaneously. The incorporation of additional peptide at ratio greater than 8 moles of peptide per mole fibrinogen (i.e. 25 moles of peptide per mole fibrinogen) might thus be useful for allowing the heparin-binding peptides to serve both as cell adhesion sites and heparin immobilization sites for drug delivery. For example, 5% of the heparin-binding sites might be used to immobilize heparin for drug delivery and the other 95% would remain unoccupied and free to serve as cell adhesion domains, thus allowing the heparin-binding peptides to be used as both cell adhesion domains and growth factor immobilization sites within the same material. This dual use of heparin-binding peptides benefits from the use of exogenous factor XIIIa because it has been shown that a relatively high number of heparin-binding sites must be incorporated into fibrin (8 moles of peptide per mole fibrinogen) in order to enhance cell adhesion and migration within fibrin matrices.

### Use of bi-domain peptides for incorporation of heparin affinity sites.

There exist many possible combinations of peptide-peptide chimeras, with a factor XIIIa substrate on one domain and a heparin-binding peptide on the other domain. These different combinations will have different advantages and disadvantages. For example, some factor XIIIa substrates are more efficiently incorporated than others. Additionally, different heparin-binding peptides have different affinity for heparin. One additional consideration is the possible immunogenicity of the peptides. Even though it is possible to utilize peptide sequences based on the sequences of the human proteins, the fusion between these two human sequences is new and would have never been seen by the immune system of a patient. As such, there exists some risk that such a fusion would be immunogenic and induce antibody formation against one or the other proteins, or against the fusion site itself. In general, shorter peptide sequences will have a lesser tendency to induce antigenic responses than longer ones. As such, the chimera with the alpha 2 plasmin inhibitor factor XIIIa substrate and the antithrombin III heparin binding domain would be somewhat more likely to induce an antigenic response than the corresponding bi-domain peptide with a heparin binding domain from platelet factor 4, for example.

An additional approach to reduce the probability than a bi-domain chimera used to immobilize heparin within the fibrin network will be immunogenic is to form a peptide-heparin chimera directly, with a factor XIIIa substrate, for example from the protein alpha 2 plasmin inhibitor, in one domain, and a heparin chain as the other domain, with a covalent bond between the two. In this manner, no non-natural peptide sequence exists at the fusion site, so the potential for immunological interactions is very low.

### Use of fusion proteins in release of proteins

One may further consider use of fusion proteins in release of proteins through affinity for heparin, in that a fusion of a bioactive protein that does not bind heparin may be constructed with a heparin-binding domain, to make a resulting fusion protein that does bind heparin. Moreover, as an alternative to the incorporation of bioactive proteins within fibrin via their affinity for heparin, the proteins may be incorporated directly using factor XIIIa. This is possible if they possess a substrate domain for factor XIIIa, either naturally or by incorporation within a recombinant protein to form a fusion protein with the bioactive protein and a factor XIIIa substrate domain.

Synthesis of either of the fusion proteins described above can be accomplished by utilizing molecular biology techniques. To do this, a fusion protein can be created that contains the entire protein sequence of interest with a cross-linking or binding sequence fused onto the amino or carboxyl terminus, or potentially elsewhere within the protein chain. This is done at the DNA level, as sequences coding for either a factor XIIIa cross-linking substrate or a heparin-binding domain can be inserted at the beginning or the end of the codons for the original protein, for example. When these modified proteins are expressed, they will then contain the additional domain of interest at the amino or carboxy terminus, or elsewhere within the main protein domain. By using the natural machinery designed for protein synthesis, it becomes possible to synthesize and purify large proteins with high fidelity.

Using standard molecular biology techniques, fusion proteins can be made of any growth factor for which the protein or DNA sequence is known, allowing the addition of novel domains such as heparin-binding domains or enzymatic substrates. These fusion proteins can be constructed so as to add a novel domain to either the N or C-terminus of the protein, for example, or within the protein chain. The modifications are made at the DNA level by constructing a gene containing both the DNA sequence coding for the growth factor and the DNA sequence coding for the crosslinking or binding sequence, for example, a heparin-binding domain. This DNA is then ligated into an expression plasmid and transformed into bacteria. Upon induction of expression, the bacteria will produce large amounts of this fusion protein. Following expression, the protein must be purified from the cell lysate and refolded. Purification is often simplified due to the tendency of mammalian proteins expressed at high level to form inclusion bodies in bacteria.

### Design of fusion proteins for incorporation

A recombinant fusion protein can be incorporated into the fibrin gels using several different schemes. In the first design, a factor XIIIa substrate has been directly incorporated onto the protein. When this modified protein is present during the polymerization of the fibrin, it is directly incorporated into the fibrin matrix in a manner similar to the bi-domain peptides. A separate method involves fusion proteins that have been synthesized to incorporate a heparin-binding domain. In this example, a bi-domain peptide, heparin, and the heparin-binding fusion protein are included in the fibrin polymerization mixture. During polymerization, the bi-domain peptide is cross-linked into the fibrin gel. This bi-domain peptide would contain a factor XIIIa substrate sequence in addition to a heparin-binding sequence. The heparin binds to the bi-domain peptide that has been incorporated in the fibrin gel and is trapped in the fibrin matrix. This entrapped heparin serves to sequester the heparin-binding fusion protein within the fibrin gel by binding to the engineered heparin-binding domains. This incorporation has been shown to be stable enough to sequester the growth factor until the cross-linked peptide is removed from the gel via cell controlled proteolysis.

According to the invention, this technique is modified by incorporating an enzymatic degradation site between the factor XIIIa substrate sequence and the protein of interest. By careful selection of Kₘ and k_{cat} of this enzymatic degradation site, degradation could be controlled to occur either before or after the protein matrix and/or by utilizing similar or dissimilar enzymes to degrade the matrix, with the placement of the degradation site being tailored for each type of protein and application. This new protein could be directly cross-linked into the fibrin matrix as described above. However, incorporating an enzymatic degradation site alters the release of the protein during proteolysis. When the cell-derived proteases reach the sequestered protein, they can cleave the engineered protein at the newly formed degradation site. The resulting degradation products would include the liberated protein, which would now be nearly free of any engineered fusion sequences, as well as any degraded fibrin. Therefore, the free protein would now be nearly identical in primary sequence to the native growth factor and potentially more bioactive. A similar method is be used with the heparin-binding fusion proteins. These new proteins would then contain the protease degradation site, as well as the new heparin-binding domain. The heparin-binding fusion proteins will be sequestered into the matrix by the incorporation of heparin into the fibrin via the covalent immobilization of heparin-binding peptides. Once again, with the new protease degradation site added, the released protein would be identical in primary sequence to the natural protein.

### III. Methods of Use

The polymers described herein can be crosslinked to form matrices for repair, regeneration, or remodeling of tissues, and/or release of bioactive factors, prior to or at the time of implantation. In some cases it will be desirable to induce crosslinking at the site of administration (not claimed) to conform the matrix to the tissue at the implantation site. In other cases, it will be convenient to prepare the matrix prior to implantation, and in the case where the matrix incorporates heparin or heparin-binding peptides which are used to bind other bioactive molecules such as growth factors, these factors may be added to the matrix prior to or at the time of implantation. It may be convenient in some cases as well to "re-fill" the matrix with these bioactive factors, where the originally loaded factors have been released.

Crosslinking can be achieved through the addition of exogenous crosslinking agent, or in the case where the polymer includes a factor XIIIa substrate, during surgical procedures or by addition of thrombin locally at the site of implantation.

Cells can also be added to the matrix prior to or at the time or implantation, or even subsequent to implantation, either at or subsequent to crosslinking of the polymer to form the matrix. This may be in addition to or in place of crosslinking the matrix to produce interstitial spacing designed to promote cell proliferation or in-growth.

Although in most cases it will be desirable to implant the matrix to promote cell growth or proliferation, in some cases the bioactive factors will be used to inhibit the rate of cell proliferation. A specific application is to inhibit the formation of adhesions following surgery.

The following examples are included to demonstrate preferred embodiments of the invention. While the compositions and methods have been described in terms of preferred embodiments, it will be apparent to those of skill in the art that variations may be applied to the composition, methods and in the steps or in the sequence of steps of the method described herein without departing from the concept, spirit and scope of the invention.

### Example 1: Indirect Couple of Heparin via a Heparin-binding Peptide to attach growth factor.

A peptide chimera containing both a factor XIIIa substrate and a heparin-binding domain was synthesized by standard solid phase synthesis. A sample peptide is one containing the following sequence, dLNQEQVSP*K( A)FAKLAARLYRKA* (SEQ ID NO:21), where the N-terminus of the peptide contains the factor XIIIa substrate and the sequence in italics contains a modified peptide from the heparin-binding domain of ATIII (dL denotes dansyl leucine, which is used to allow detection of the peptide by fluorescence).

Size exclusion chromatography was used to determine the amount of peptide cross-linked to fibrin gels using the previously developed incorporation method. A bi-domain peptide containing the heparin-binding domain from antithrombin III and a fluorescent label was incorporated into fibrin gels during polymerization. The free peptide was washed from the gels, and the fibrin network was degraded with plasmin. The degradation products were analyzed by high performance liquid chromatography (size exclusion chromatography) to determine the amount of peptide (by fluorescence) present per mole of fibrinogen (by UV absorbance). The fluorescence signal from peptide-modified gels appeared at an earlier elution time than did the signal from free peptide alone, indicating that all peptide present in the modified gels was cross-linked to fibrin (FIGURE 1). Quantification based on standards of known concentration for both peptide and fibrin networks degraded with plasmin showed incorporation of 8.7 ± 0.2 moles of peptide per mole of fibrinogen (n=10.

In order to evaluate this approach to release of heparin-binding growth factors from fibrin cell in-growth matrices that also comprise a covalently linked bi-domain peptide, one domain of which is a factor XIIIa substrate and one domain of which is a heparin-binding domain based on antithrombin III, dorsal root ganglia were cultured three-dimensionally within fibrin gels under a variety of conditions, as shown in the below.

### Cross-linking Protocol for use of Heparin-Binding Peptides:

1) Dialyze fibrinogen (8 mg/ml) versus 4 L of Tris buffered saline (33 mM Tris), pH 7.4 for 24 hours.
2) Sterile filter fibrinogen using a 0.2 µm syringe filter.
3) Make the following peptide solutions:

| | Peptide (25 mg/ml) | Heparin (45 mg/ml) | BFGF (5 µg/ml) | Tris buffered saline (TBS) |
|---|---|---|---|---|
| Fibrin | 0 µl | 0 µl | 0 µl | 980 µl |
| Peptide | 70 µl | 0 µl | 0 µl | 910 µl |
| Peptide + heparin | 70µl | 70 µl | 0 µl | 840 µl |
| Peptide + heparin + bFGF | 70µl | 70µl | 56µl | 784 µl |

4) Make thrombin solution: 100 units in 5 ml TBS.
5) Add 1.4 ml of fibrinogen to each peptide solution.
6) Make gels: Add 20 µl of TBS + 50 mM CaCl₂, 40 µl of thrombin solution (20 units/ml), and 340 µl of peptide solution + fibrinogen. (above solutions make 6 gels).
7) Incubate at 37°C for 1 hr.
8) Wash 5 times in 24 hours. Use 1 ml of TBS the first 4 times and neuronal media the last time.
9) Dissect day 8 chick embryonic dorsal root ganglia.
10) Place one ganglia in each gel and place at 37°C for 1 hr.
11) Add 1 ml of neuronal media to each gel.
12) Change media after 24 hours.

The results of these studies with dorsal root ganglion culture are shown in Figure 2.
These results show that the heparin and peptide alone do not increase neurite extension. When added without peptide and heparin, bFGF does not enhance neurite outgrowth, demonstrating that the washing protocol used is sufficient. Neurite enhancement is increased by the addition of both 1 µg/ml and 5 µg/ml of bound bFGF in a dose dependent manner. The addition of 1.0 µg/ml bound VEGF did not increase neurite extension, suggesting that the effect bFGF is not due to its ability to promote angiogenesis.

### Example 2: Synthesis of Heparin-Peptide Chimeras

A heparin-peptide chimera is synthesized by coupling a peptide, containing the factor XIIIa substrate on the N-terminus and a poly-lysine on the C-terminus, to a heparin oligosaccharide, with a unique aldehyde group on one end, via reductive amination. A peptide with the following sequence, dLNQEQVSPLKKKG (SEQ ID NO:22), is synthesized by standard solid phase peptide chemistry. The heparin oligosaccharides are made by standard nitrous acid degradation of heparin, resulting in the formation of an aldehyde on the reducing terminal of the cleaved oligosaccharide. During coupling, the -amino group of the lysine side chain attacks the aldehyde on the reducing end of the heparin oligosaccharide to form a Schiff base. The Schiff base is then reduced to form a stable product. A sample coupling protocol is given below. One can also couple the heparin to the simpler alpha 2 plasmin inhibitor substrate site NQEQVSP (SEQ ID NO:20). A primary amine exists on this peptide only at the N-terminus. When the peptide is synthesized, as usual, on a solid phase resin, the N-terminus is exposed dangling, and when the alpha amine on the terminal N is deprotected, a primary amine is available for reaction. A reactive form of heparin may be readily formed by cleavage in certain acids, as described in Grainger, D., et al., (1988). J. Biomed Mat. Res. 22:231-249, to form a heparin fragment with a terminal aldehyde group. This reactive aldehyde can be passed over the peptide still attached to the resin, condensed there upon, to form a peptide-heparin chimera that is linked by a Schiff base, which may be readily reduced with sodium cyanoborohydride to form a secondary amine, which is a more stable form.

Other approaches could be used. For example, the aldehyde heparin may be converted to a primary amino heparin by reaction with excess ethylene diamine and reduction of the Schiff base, analogous to that described above. Purification from the free residual ethylene diamine can be achieved by dialysis. This amino heparin may be condensed on the C-terminal carboxyl group by cleaving the peptide from the solid resin with the carboxyl group on the E residue still protected, followed by activation of the carboxyl group on the C terminus using standard reagents from peptide synthesis, and then followed by deprotection of the carboxyl group on the E residue.

### Coupling Protocol:

1) Dissolve 1.8 mM of peptide and 1.8 mM of nitrous acid degraded heparin in 50 mM borate buffer, pH 9. React for 30 minutes.
2) Add 160 mM NaCNBH₃ and react for 12 hours.
3) Add 240 mM NaCNBH₃ aud react for 12 hours.
4) Adjust pH to 7 with dilute HCl.
5) Add NaCl to a final concentration of 1M.
6) Dialyze versus 4L of deionized water for 24 hours.
7) Lyophilize to obtain reaction product.
8) Analyze reaction yield by size exclusion chromatography.
9) Purification of desired product is accomplished using anion exchange chromatography.

### Use: Cross-linking Protocol for use of Heparin-Peptide Chimeras:

1) Dialyze fibrinogen (8 mg/ml) versus 4 L of Tris buffered saline (33 mM Tris), pH 7.4 for 24 hours.
2) Sterile filter fibrinogen using a 0.2 µm syringe filter.
3) Make the following chimera solutions:

| | heparin-peptide chimera (67 mg/ml) | bFGF (5 µg/ml) | Tris buffered saline (TBS) |
|---|---|---|---|
| Fibrin | 0 µl | 0 µl | 980 µl |
| heparin-peptide chimera | 70µl | 0 µl | 940 µl |
| heparin-peptide chimera + bFGF | 70µl | 56µl | 784 µl |

4) Make thrombin solution: 100 units in 5 ml TBS.
5) Add 1.4 ml of fibrinogen to each chimera solution.
6) Make gels: Add 20 µl of TBS + 50 mM CaCl₂, 40 µl of thrombin solution (20 units/ml), and 340 µl of chimera solution + fibrinogen (above solutions make 6 gels).
7) Incubate at 37°C for 1 hr.
8) Wash 5 times in 24 hours. Use 1 ml of TBS the first 4 times and neuronal media the last time.
9) Dissect day 8 chick embryonic dorsal root ganglia.
10) Place one ganglia in each gel and place at 37C for 1 hr.
11) Add 1 ml of neuronal media to each gel.
12) Change media after 24 hours.

### Example 3: Degradable Sites in Fusion Protein and In Peptide Chimera

### NGF fusion proteins containing Factor XIIIa substrate and a plasmin degradation site

β-NGF fusion proteins were expressed with an exogenous cross-linking substrate that allows the β-NGF fusion proteins to be enzymatically cross-linked to fibrin matrices, which served as the base material for the drug delivery system. A plasmin substrate was placed between the cross-linking substrate and the β-NGF domain in the fusion protein, which served as a degradable linker and allowed β-NGF to be released from the matrix in a form almost identical to its native sequence by enzymatic cleavage. The β-NGF fusion proteins were covalently attached to fibrin by the transglutaminase activity of factor XIIIa and were tested in an *in vitro* model of nerve regeneration to determine the ability of the delivery system to release active growth factors in response to cell-associated enzymatic activity.

### Gene synthesis

Two β-NGF fusion proteins were made by recombinant protein expression. Each protein contained a cross-linking substrate at the N-terminus of the protein that consisted of the transglutaminase (TG) factor XIIIa substrate from α₂-plasmin inhibitor, NQEQVSPL (SEQ ID NO:23). Each β-NGF fusion protein also contained the native β-NGF sequence at the C-terminus of the protein. One of two plasmin substrates (P) was placed between the cross-linking substrate and the β-NGF domain of the fusion protein, either a functional plasmin substrate (LIK/MKP, where / denotes the cleavage site) or a non-functional plasmin substrate (LINMKP) in which the lysine residue at the cleavage site in the plasmin substrate was changed to an asparigine residue to render the plasmin substrate non-functional. The fusion protein containing a functional plasmin substrate was denoted TG-P-NGF, and the fusion protein containing a non-functional plasmin substrate was denoted TG-Pᵢ-NGF.

The protein sequence to expressed is as follows: *MGSSHHHHHHSSGLVPRGS*HM**NQEQVSPLP**VELPLIKMKPVELESSS HPIFHRGEFSVCDSVSVWVGDKTTATDIKGKEVMVLGEVNINNSVFK QYFFETKCRDPNPVDSGCRGIDSKHWNSYCTTTHTFVKALTMDGKQ AAWRFIRIDTACVCVLSRKAVRZ (SEQ ID NO:24), where the region in italics is the Histidine tag derived from the expression vector, and the underlined region is the thrombin cleavage site. The residues are the cross-linking substrate sequence for factor XIIIa, and double underlined region denotes the plasmin substrate.

The cloning plasmid used for gene assembly was pUC 18. The DNA sequence of the gene is as follows from 5' to 3':

GAATTCCCATGGCATATGAACCAGGAACAGGTTAGCCCGCTGCCC
GTGGAACTGCCGCTGATCAAAATGAAACCCGTGGAACTCGAGAG
CTCTTCCCACCCGATTTTCCATCGTGGCGAGTTCTCCGTGTGTGAC
TCTGTCTCTGTATGGGTAGGCGATAAAACCACTGCCACTGATATC
AAAGGCAAAGAGGTGATGGTGCTGGGAGAAGTAAACATTAACAA
C[?]TCTGTATTCAAACAGTACTTCTTCGAAACTAAGTGCCGTGACCC
GAACCCGGTAGACTCTGGGTGTCGCGGCATCGATTCTAAACACTG
GAACTCTTACTGCACCACTACTCACACTTTCGTTAAAGCGTTGACT
ATGGATGGTAAACAGGCTGCCTGGCGTTTCATCCGTATCGATACT
GCATGCGTGTGTGTACTGTCCCGTAAAGCTGTTCGTTAAGGATCC
(SEQ ID NO:25).

In order to synthesize the β-NGF fusion protein by recombinant protein expression, the gene coding for the protein was cloned. Once the gene fragments were designed, Eco RI and Hind III sites were added to the end of each fragment, to allow the cloning of these fragments into the poly-cloning linker of pUC18 (Gibco, Basel, Switzerland). The 3' and 5' single-stranded oligonucleotides of each gene fragment were synthesized by Microsynth (Balgach, Switzerland) with sticky ends for the two cloning restriction sites. The single-stranded oligonucleotides were purified using denaturing poly-acrylamide gel electrophoresis (PAGE), the highest molecular weight band for each fragment was extracted from the gel, and the corresponding 3' and 5' oligonucleotide fragments were annealed. The annealed fragments were phosphorylated with T4 DNA kinase (Boehringer Mannheim, Rotkreuz, Switzerland) and ligated into pUC18. Following ligation, the plasmids were transformed into DH5 -F' competent cells and plated on isopropyl -D-thiogalactopyranoside (IPTG)/ 5-bromo-4chloro-3-indolyl -D-galactopyranoside (X-gal)/ampicillin (Amp) plates to screen for insertion of the gene fragments into the plasmid. Plasmids from colonies containing inserted gene fragments were sequenced to identify colonies containing gene fragments with the correct sequence. After the correct sequence for each gene fragments was obtained, the fragments were assembled to form the complete gene for the fusion protein. Briefly, plasmids that contained fragment 2 were digested with the enzymes EcoR V and Hind III (Boehringer Manuheim), and the fragments were purified by non-denaturing PAGE. Plasmids containing fragment 1 were digested with the enzymes Eco RV and Hind III, dephosphorylated with alkaline phosphatase (Boehringer Mannheim), and the digested plasmids were purified by agarose gel electrophoresis. Fragment 2 was ligated into the digested plasmids that contained fragment 1 to obtain a plasmid that contained both fragments 1 and 2. This plasmid was transformed into DH5 - F' competent cells, and plated on Amp plates. The resulting colonies were screened for plasmids containing both fragments, and these plasmids were sequenced. This process was repeated until the complete gene for the β-NGF fusion protein was assembled.

The gene for the TG-Pᵢ-NGF fusion protein was made from the TG-P-NGF gene by site directed mutagenesis. Polymerase chain reaction (PCR) was performed to modify the region of the fusion protein gene coding for the plasmin substrate, using primers containing the desired modification of the gene. Using the TG-P-NGF gene as a template, two reactions were performed, one with primer A and primer B, and the other with primer C and primer D.
Primer A AACAGCTATG ACCATG (M13 reverse)
Primer B GTTTCATGTT GATCAGCGGC AGT
Primer C TGATCAACAT GAAACCCGTG GAA
Primer D GTAAAACGACG GCCAGT (M13) (SEQ ID NO:26-29) The products from the two reactions were purified by agarose gel electrophoresis, and used as primers for the third reaction. Primers A and D were also added to the third reaction to amply the desired product. The final reaction product was digested with Eco RI and Hind III and purified by agarose gel electrophoresis. The PCR fragment was cloned into pUC18, and sequenced to identify the correct PCR product.

### Protein Expression

The complete gene for each of the β-NGF fusion proteins was digested out of pUC18 and ligated into the expression vector, pET14b (Novagen, Madison, Wisconsin). The expression vector was transformed into the expression host, BL21 (DE3) pLysS to allow for tight regulation of fusion protein expression. The fusion protein was expressed by growing the *E. coli* until they reached mid-log phase growth (optical density at 600 nm of 0.4-0.6) and then inducing protein expression by the addition of 0.4 mM IPTG to the culture medium. The bacteria were harvested after 2 hr by centrifugation at 5500xg. After harvesting, the cells were suspended in 1/10 the culture volume of 20 mM Tris HCl, 250 mM NaCl, pH 8.0. Lysozyme (0.4 mg/mL) and DNase (5 ng/mL) were added to the harvested cells, and the solution was incubated at 37°C for 30 minutes. The inclusion bodies were collected from the cell lysate by centrifugation at 10,000 x g for 15 min. The pellet containing the inclusion bodies was resuspended in 40 mL/ liter culture volume of binding buffer (5 mM imidazole, 0.5 M NaCl, 20 mM Tris HCl, pH 7.9) containing 6 M guanidine hydrochloride (GuHCl) at room temperature for 90 min. Insoluble material in the solution was collected by centrifugation for 20 min at 20,000 x g, and the supernatant, which contained the solubilized fusion protein, was saved for further purification.

### Protein Purification

The fusion protein contained a thrombin-cleavable histidine tag for purification at the N-terminus of the protein, because the gene for the β-NGF fusion protein was inserted in pET14b between the Nde I and Bam HI sites. Nickel affinity chromatography was used to purify the β-NGF fusion protein. His Bind™ resin (Novagen) was packed into a chromatography column (2.5 mL bed volume per liter culture volume), charged with Ni⁺⁺ and equilibrated with binding buffer containing 6 M GuHCl (according to the manufacturer's instructions). The supernatant, which contained the fusion protein, was filtered with a 5 µm syringe filter and loaded on the column. The column was washed with 10 column volumes of binding buffer containing 8 M urea and 6 column volumes of wash buffer (20 mM imidazole, 0.5 M NaCl, 20 mM Tris HCl, pH 7.9) containing 8 M urea. The fusion protein was eluted with 4 column volumes of elution buffer (1 M imidazole, 0.5 M NaCl, 20 mM Tris HCl, pH 7.9) containing 8 M urea. The presence of β-NGF fusion protein in the elution fractions was confirmed by sodium dodecyl sulfate (SDS)-PAGE.

### Protein Refolding

The β-NGF fusion protein was refolded by adding a 5-fold excess of ice cold refolding buffer (20 mM Tris HCl, 250 mM NaCl, 2 mM reduced glutathione and 0.2 mM oxidized glutathione, pH 8.0) to the purified β-NGF fusion protein slowly, until a final urea concentration of 1.3 M was attained. The fusion protein was refolded for 48 hr at 4°C while stirring. The refolded fusion protein was dialyzed against a 50-fold excess of storage buffer (20 mM Tris HCl, 500 mM NaCl, pH 8.0) containing 10% glycerol at 4°C overnight The fusion protein was concentrated by centrifugation using Vivaspin^{™} concentrators (5000 MW cutoff, Vivascience, Lincoln, UK) to a concentration of about 300-400 µg/mL, as measured by Bradford assay.

### Incorporation of β-NGF Fusion Protein into Fibrin Matrices

To determine the efficiency of β-NGF fusion protein incorporation into fibrin matrices, TG-Pᵢ-NGF fusion protein was labeled with biotin to allow β-NGF quantification by a direct enzyme-linked immunosorbent assay (ELISA). A 20-fold molar excess of Sulfo-N-hydroxysuccinimide (NHS)-LC Biotin (Pierce, Lausanne, Switzerland) was added to the β-NGF fusion protein in phosphate buffered saline (PBS, 0.01 M phosphate buffer, 8 g/L NaCl, 0.2 g/L KCl, pH 7.4) at a concentration of 1 mg/mL from a stock solution of 10 mg/mL biotin in N,N-dimethylformamide (dissolved for 10. min at 37°C). The reaction was allowed to proceed for 2 hr at room temperature. The unreacted biotin was then removed by gel nitration chromatography using a PD-10 column (Amersham Pharmacia, Dubendorf, Switzerland).

A known amount of labeled β-NGF was adsorbed to 96 well plates overnight in coating buffer (0.1 M NaHCO₃, pH 8) at 4°C. The wells were blocked with 1% bovine serum albumin (BSA) in PBS for 2 hr at room temperature. The wells were washed 3 times in PBS with 0.5% Tween-20 (PBST buffer). Horse radish peroxidase (HRP)-conjugated streptavidin was diluted to 1 µg/mL in PBS and added to each well for 1 hr. The wells were washed 3 times with PBST buffer and then incubated in ABTS (2,2'-Azino-bis(3-ethylbenz-thiazoline-6-sulfonic acid)) developing solution (0.1M NaC₂H₃O₂, 0.05 MNaH₂PO₄, 0.1% ABTS, 0.01% H₂O₂, pH 42). After 1-5 minutes, the reaction was stopped by adding an equal volume of 0.6% SDS, and the absorbance of each well was measured at 405 nm using an EL311SX plate reader from Bio-Tek Instruments (Winooski, Vermont). A standard curve of β-NGF concentration versus absorbance at 405 nm was made using these measurements from the direct ELISA assay.

Plasminogen-free fibrinogen was dissolved in water and dialyzed versus Tris-buffered saline (TBS, 33 mM Tris, 8 g/L NaCl, 0.2 g/L KCl) at pH 7.4 for 24 hr. β-NGF fusion protein was incubated with 5 mM Ca⁺⁺ and 4 NIH units/mL thrombin for 1 hr at 37°C to remove the histidine tag used for purification. The β-NGF fusion protein solution was mixed in equal ratio with fibrinogen at a concentration of 8 mg/mL and polymerized at 37°C for 60 min. The fibrin matrices were washed 5 times over 24 hr, and each wash was saved to determine the total amount of β-NGF washed out of the matrix. After 24 hr, the fibrin matrices containing β-NGF were degraded with 0.1 U of porcine plasmin. The amount of β-NGF in the washes and remaining in the matrix was quantified as described above by direct ELISA and a β-NGF standard curve was constructed for each ELISA performed.

A Western blot was performed to show directly that β-NGF fusion proteins were covalently coupled to fibrin matrices. Fibrin matrices were made and washed as described in the incorporation quantification assay. The matrices were washed 5 times over 24 hr and then degraded with plasmin, as described above. The degradation products were separated by SDS-PAGE using a 13.5% denaturing geL The proteins from the gel were transferred to an activated Immobilon-P^{™} polyvinylidene difluoride (PVDF) membrane (Millipore, Volketswil, Switzerland) with a current of 400 mA for 1 hr. The membrane was dried overnight The proteins transferred to the membrane, including the molecular weight marker, were visualized by staining with 0.2% Ponceau S. Non-specific protein binding to the membrane was blocked with 3% BSA in TBS for 2 hr. The membrane was incubated with goat antihuman β-NGF antibody (R&D Systems, Minneapolis, Minnesota) at a concentration of 0.2 µg/mL in 3% BSA for 1 hr. The membrane was washed 3 times with TBS for 5 min and incubated with the secondary antibody, HRP-conjugated rabbit anti-goat immunoglobins (Dako Diagnostics, Zug, Switzerland) at a concentration of 0.5 µg/mL in 3% BSA for 30 min. The membrane was washed 3 times with TBS, and then incubated for 5 min with an enhanced chemi-luminescent HRP substrate (Pierce, Lausanne, Switzerland) diluted 1:5 in TBS. Excess liquid was removed from the membrane and it was covered with plastic and was exposed to X-ray film for 5-60 sec.

An increase in molecular weight was indeed observed for β-NGF fusion proteins that were present during polymerization of fibrin matrices), while in the case of β-NGF lacking a cross-linking substrate, no β-NGF was observed in the matrix after washing. This result showed directly that the β-NGF fusion protein was covalently immobilized within fibrin matrices during polymerization via the transglutaminase activity of factor XIIIa.

### Bioactivity of Immobilized β-NGF Fusion Protein

To determine the ability of covalently immobilized β-NGF fusion protein to be delivered in a controlled manner, β-NGF fusion proteins were incorporated into fibrin matrices during polymerization, and the ability of these matrices to enhance neurite extension *in vitro* was assayed using chick DRGs. TG-P-NGF was found to enhance neurite extension by over 350% versus unmodified fibrin with no NGF present in the culture medium and by up to 50% over unmodified fibrin with 10 ng/mL of native NGF in medium (Figure 3). TG-Pᵢ-NGF, which contained a non-functional plasmin substrate, could not be cleaved from the fibrin matrix by plasmin in a native form and did not significantly enhance neurite extension versus native NGF in the culture medium, when covalently immobilized within fibrin matrices at any of the concentrations tested. However, TG-P-NGF, which contained a functional plasmin substrate, could be cleaved from the matrix by plasmin in a form very similar to native NGF and was observed to enhance neurite extension, even when compared with similar doses of native NGF present in the culture medium. A dose response effect for TG-P-NGF fusion protein was observed, with an optimal dose attained when 1-5 µg/mL of β-NGF fusion protein was present in the polymerization mixture. These results demonstrated that the TG-P-NGF fusion protein was bioactive when immobilized within fibrin matrices, suggesting that it could be released in an active form by cell-associated matrix degradation. Despite the lower activity of the β-NGF fusion proteins in the PC12 cell activity assay, when the plasmin degradable β-NGF fusion protein was covalently coupled to fibrin, it promoted greater levels of neurite extension than the same dose of native NGF in the culture medium. These results also suggested that for the β-NGF fusion proteins to be fully active, they must be release from the fibrin matrix in a form similar to that of their native structure.

### Efficiency of β-NGF Fusion Protein Cross-linking

To determine the efficiency of β-NGF fusion protein incorporation in fibrin matrices, the protein was labeled with biotin, and a direct ELISA was performed on fibrin matrices that contained biotin-labeled β-NGF fusion protein in the polymerization mixture. Biotin-labeled β-NGF fusion proteins were incorporated into fibrin matrices during polymerization. After washing to remove any unbound β-NGF fusion protein, the matrices were degraded with plasmin and the amount of β-NGF in the degraded matrices and in the washes was quantified. The percentage of β-NGF fusion protein incorporated into the fibrin matrix is shown in Figure 4 as a function of β-NGF concentration in the polymerization mixture. Over the range β-NGF fusion protein concentrations tested, 50-60% of the fusion protein was incorporated during polymerization of the fibrin matrix. This result demonstrated that β-NGF fusion proteins were incorporated into fibrin matrices efficiently through the action of factor XIIIa.

### β-NGF fusion protein with a heparin binding domain and a plasmin degradation site

NGF can be expressed as fusion protein in *E. coli*, which contains a heparin-binding domain at the N-terminus, a plasmin substrate in the middle and the NGF sequence at the C-terminus of the protein. This is accomplished by constructing a synthetic gene containing the DNA which codes for the desired fusion protein. The protein sequence to expressed is as follows:
*MGSSSHHHHHHSSGLVPRGS*HMKDPKRLYRSRKLPVELPLIKMKPVEL ESSSHPIFHRGEFSVCDSVSVWVGDKTTATDIKGKEVMVLGEVNINN SVFKQYFFETKCRDPNPVDSGCRGIDSKHWNSYCTTTHTFVKALTM DGKQAAWRFIRIDTACVCVLSRKAVRZ (SEQ ID NO:30), where the region in italics is the Histidine tag derived from the expression vector, and the underlined region is the thrombin cleavage site. Dotted underline denote the heparin-binding sequence, and double underline denotes the plasmin substrate.

The cloning plasmid used for gene assembly was pUC 18.. The DNA sequence of the gene is as follows from 5' to 3':

GAATTCCCATGGCATATGAAAGACCCGAAACGTCTGTACCGTTCT
CGTAAACTGCCCGTGGAACTGCCGCTGATCAAAATGAAACCCGTG
GAACTCGAGAGCTCTTCCCACCCGATTTTCCATCGTGGCGAGTTCT
CCGTGTGTGACTCTGTCTCTGTATGGGTAGGCGATAAAACCACTG
CCACTGATATCAAAGGCAAAGAGGTGATGGTGCTGGGAGAAGTA
AACATTAACAACTCTGTATTCAAACAGTACTTCTTCGAAACTAAG
TGCCGTGACCCGAACCCGGTAGACTCTGGGTGTCGCGGCATCGAT

TCTAAACACTGGAACTCTTACTGCACCACTACTCACACTTTCGTTA
AAGCGTTGACTATGGATGGTAAACAGGCTGCCTGGCGTTTCATCC
GTATCGATACTGCATGCGTGTGTGTACTGTCCCGTAAAGCTGTTCG
TTAAGGATCC (SEQ ID NO:31).

This gene is inserted between the EcoRI and HindIII sites in the polylinker cloning region of pUC 18, as shown in the map.

After assembly this gene is inserted into the expression vector. Expression and purification are then performed as described above.

### Example 4: Fusion Proteins of growth factors that do bind heparin spontaneously.

### A. Biosynthesis of Factor XIIIa substrate fusion proteins with NGF

NGF can be expressed as fusion protein in *E. coli*, which contains a factor XIIIa substrate at the N-terminus and the human β-NGF sequence at the C-terminus of the protein. This is accomplished by constructing a synthetic gene containing the DNA which codes for the desired fusion protein. The protein sequence to expressed is as follows:
*MGSSHHHHHHSSGLVPRGS*HM**NQEQVSPLP**VELESSSHPIFHRGEFSV CDSVSVWVGDKTTATDIKGKEVMVLGEVNINNSVFKQYFFETKCRD PNPVDSGCRGIDSKHWNSYCTTTHTFALTMDGKQAAWRFIRIDT ACVCVLSRKAVRZ (SEQ ID NO:32), where the region in italics is the Histidine tag derived from the expression vector, and the underlined region is the thrombin cleavage site. The residues are the cross-linking substrate sequence for factor XIIIa.

The cloning plasmid used for gene assembly was pUC 18, which is the same as pUC 19 except that the sequence of the polylinker cloning region is reversed. A map of pUC 19 follows, which was obtained from New England Biolabs. The DNA sequence of the gene is as follows from 5' to 3':

GAATTCCATATGAACCAGGAACAGGTTAGCCCGCTGCCCGTGGAA
CTCGAGAGCTCTTCCCACCCGATTTTCCATCGTGGCGAGTTCTCCG
TGTGTGACTCTGTCTCTGTATGGGTAGGCGATAAAACCACTGCCA
CTGATATCAAAGGCAAAGAGGTGATGGTGCTGGGAGAAGTAAAC

ATTAACAACTCTGTATTCAAACAGTACTTCTTCGAAACTAAGTGC
CGTGACCCGAACCCGGTAGACTCTGGGTGTCGCGGCATCGATTCT
AAACACTGGAACTCTTACTGCACCACTACTCACACTTTCGTTAAA
GCGTTGACTATGGATGGTAAACAGGCTGCCTGGCGTTTCATCCGT
ATCGATACTGCATGCGTGTGTGTACTGTCCCGTAAAGCTGTTCGTT
AAGGATCC (SEQ ID NO:33).

This gene is inserted between the EcoRI and HindIII sites in the polylinker cloning region of pUC 18, as shown in the map. After gene assembly, this gene is inserted into the expression vector pET 14b between the NdeI and BamHI sites. A map of the pET 14b vector follows, which was obtained from Novagen. After insertion of the gene into the expression vector, the plasmid is transformed into BL21(DE3)pLysS competent cells. The cell are grown until they reach an OD of about 0.6, then they are induced to express of the fusion protein with IPTG (final concentration in solution 0.4 mM). Expression is continued for 2-3 hours. The cells are placed on ice for 5 minutes and then harvested by centrifugation at 5000 x g for 5 min. at 4°C. They are resuspended in 0.25 culture volume of cold 50 mM Tris-HCl pH 8.0 at 25C. The cells are centrifuged as before and the pellet is frozen. Cells are lysed upon thawing.

The cell lysate is centrifuged and the supernatant discarded. The pellet is resuspended in Triton X100. The solution is then centrifuged and the supernatant is discarded. The pellet is resuspended in 6M urea and the fusion protein is purified by histidine affinity chromatography. The histidine tag can be cleaved by thrombin during polymerization and washed from the gels during the standard washing procedure.

### B. Biosynthesis of heparin-binding domain fusion proteins of NGF

NGF can be expressed as fusion protein in *E. coli*, which contains a heparin-binding domain at the N-terminus and the NGF sequence at the C-terminus of the protein. This is accomplished by constructing a synthetic gene containing the DNA which codes for the desired fusion protein. The protein sequence to expressed is as follows: *MGSSHHHHHHSSGLVPRGS*HMKDPKRLYRSRKLPVELESSSHPIFHRG EFSVCDSVSVWVGDKTTATDIKGKEVMVLGEVNINNSVFKQYFFET KCRDPNPVDSGCRGIDSKHWNSYCTTTHTFVKALTMDGKQAAWRFI RIDTACVCVLSRKAVRZ (SEQ ID NO:34), where the region in italics is the Histidine tag derived from the expression vector, and the underlined region is the thrombin cleavage site. The region underlined with a dotted underline is the heparin-binding sequence.

The cloning plasmid used for gene assembly was pUC 18. The DNA sequence of the gene is as follows from 5' to 3':

GAATTCCCATGGCATATGAAAGACCCGAAACGTCTGTACCGTTCT
CGTAAACTGCCCGTGGAACTCGAGAGCTCTTCCCACCCGATTTTC
CATCGTGGCGAGTTCTCCGTGTGTGACTCTGTCTCTGTATGGGTAG
GCGATAAAACCACTGCCACTGATATCAAAGGCAAAGAGGTGATG
GTGCTGGGAGAAGTAAACATTAACAACTCTGTATTCAAACAGTAC
TTCTTCGAAACTAAGTGCCGTGACCCGAACCCGGTAGACTCTGGG
TGTCGCGGCATCGATTCTAAACACTGGAACTCTTACTGCACCACT
ACTCACACTTTCGTTAAAGCGTTGACTATGGATGGTAAACAGGCT
GCCTGGCGTTTCATCCGTATCGATACTGCATGCGTGTGTGTACTGT
CCCGTAAAGCTGTTCGTTAAGGATCC (SEQ ID NO:35).

This gene is inserted between the EcoRI and HindIII sites in the polylinker cloning region of pUC 18, as shown in the map. After assembly this gene is inserted into the expression vector. Expression and purification are then performed as described above.

### EXAMPLE 5: BMP2 Delivery from Fibrin matrices with Bi-domain peptides for Heparin Affinity.

It has been demonstrated that bone morphogenetic factor bound to the fibrin matrix is able to control the release of this factor and when this matrix is implanted subcutaneously ectopic bone is formed. While this procedure is different from bone formation within a bony defect, it does provide a suitable method for testing the ability to control the release of a bioactive growth factor *in vivo*.

Fibrin gels were synthesized with a different bi-domain peptide with the sequence LNQEQVSPK( A)FAKLAARLYRKA (SEQ ID NO:36). This peptide is derived from the Factor XIIIa substrate sequence for α₂-plasmin inhibitor in the first domain and a mimetic of the heparin binding domain of antithrombin III in the second domain. BMP-2 is a heparin binding protein and will bind to the sequence given above. Gels were polymerized in the presence of the peptide (at 1 mM), heparin and recombinant human BMP-2 in ratios of heparin to BMP-2 of 1:1 and 40:1.

Control gels were created that had equivalent amounts of the morphogenetic protein, BMP-2, but lacking the heparin and bi-domain peptide. These gels were then implanted subcutaneously into rats and were allowed to remain for two weeks. When the matrices were extracted little to no bone was observed from the gels that did not contain the peptide-heparin release system while the gels that did contain the system showed significant bone formation.

This is best seen in the mass of the removed matrices which is shown in Table 3.

| Treatment | Explanted Matrix (mg ± s.e.m.) |
|---|---|
| Fibrin + 20 microgram BMP-2 | 7.4 ± 6.5 |
| Fibrin + 1 mMol peptide + 26 microgram heparin + 20 microgram BMP-2 | 34.5 ± 25.5 |
| Fibrin + 1 mMol peptide + 1 mg heparin + 20 microgram BMP-2 | 39.2 ± 20.8 |

The release system enhanced the formation of ectopic bone within the matrix, demonstrating the viability of the release system *in vivo*.

### Example 6: Incorporation of Peptide with exogenous Factor XIII.

Purified fibrinogen as obtained via standard precipitation methods contains small amounts of endogenous factor XIIIa which act as a limiting reagent in the incorporation of the bi-domain peptide. This was demonstrated using purified fibrinogen, in which it was possible to incorporate the bi-domain peptides at concentrations up to 8.2 mol peptide/mol fibrinogen based on the endogenous factor XIIIa concentration. Addition of exogenous factor XIIIa was demonstrated to enhance the level of peptide incorporation. This is particularly relevant for control of the bi-domain peptide concentration within the gel, which in turn will affect both cell adhesion as well as determine the upper limit for heparin and growth factor incorporation. In some cases it may be advantageous to increase cellular adhesion, heparin, or growth factor concentrations beyond that possible with standard purified fibrinogen.

Exogenous factor XIII, purified from pooled plasma, was used to incorporate the bidomain peptide, dLNQEQVSPLRGD (SEQ ID NO:1 ), into the gels. 1 U of exogenous factor XIII was added per mL fibrin gel, and the level of covalently incorporated bi-domain peptide analyzed through chromatographic analysis. The results are shown in Figure 5. When I U/mL of exogenous Factor XIII was added the level of incorporation reaching 25 mol peptide/mol fibrinogen. This level of incorporation is close to the theoretical limit based on the number of possible binding sites

The ability of the bi-domain peptide, dLNQEQVSPLRGD (SEQ ID NO:1), to incorporate into commercially available fibrin glue kits was also demonstrated. Tissucol kits were obtained and then fractionated into multiple samples. Exogenous bi-domain peptide was added at up to 6 mM and the level of incorporation was measured through chromatographic analysis (Figure 6). The level of peptide incorporation was tested over a wide range of initial bi-domain peptide concentrations for three separate kits. When the level of incorporation was measured, it was observed that the maximal incorporation occurred at concentrations of greater than 5 mM exogenous peptide. It may be that in these highly dense matrices, diffusion begins to play a role in the process of incorporation. However, a very high level of incorporation was observed with the level reaching at least 25 mol peptide/mol fibrinogen. Furthermore, there is a significant variability in the composition of fibrin glue kits with a wide range of possible protein concentrations present. However, this clearly did not affect the incorporation of the bi-domain peptide significantly as the incorporation profile was similar for all three kits tested.

## Claims

1. A crosslinked matrix suitable for cellular growth or in-growth, wherein at least one engineered protein or polysaccharide graft is covalently or ionically linked to the matrix,
wherein the engineered protein or polysaccharide graft comprises
a first domain comprising a bioactive factor or a polysaccharide,
a second domain, wherein the engineered protein or polysaccharide graft is linked to the matrix by the second domain, and
an enzymatic or hydrolytic degradation site between the first domain and the second domain.

2. The matrix of claim 1, wherein the bioactive factor is a peptide.

3. The matrix of claim 2, wherein the peptide comprises at least one heparin-binding domain.

4. The matrix of claim 1, wherein the bioactive factor is a growth factor or a protein.

5. The matrix of claim 4, wherein the bioactive factor is a heparin-binding growth factor.

6. The matrix of claim 5, wherein the heparin-binding growth factor is selected from the group consisting of transforming growth factor-beta, bone morphogenic proteins, fibroblast growth factor, vascular epithelial growth factor, interleukin-8, neurotrophin-6, heparin-binding epidermal growth factor, hepatocyte growth factor, connective tissue growth factor, midkine, heparin-binding growth associated molecules and mixtures thereof.

7. The matrix of claim 1, wherein the bioactive factor is an enzyme.

8. The matrix of claim 1, wherein the polysaccharide is selected from the group consisting of heparin, heparin sulfate and chondroitin sulfate.

9. The matrix of any of the claims 1 to 8, wherein the matrix is selected from the group consisting of proteins, polysaccharide, glycoproteins, and synthetic materials.

10. The matrix of claim 9, wherein the matrix comprises fibrin.

11. The matrix of claim 1, wherein the degradation site is an enzymatic degradation site, which is cleaved by an enzyme selected from the group consisting of plasmin and matrix metalloproteinase.

12. The matrix of any one of claims 1 to 11, wherein the second domain is a substrate domain for a crosslinking enzyme.

13. The matrix of claim 12, wherein the second domain comprises a transglutaminase substrate domain.

14. The matrix of claim 13, wherein the second domain is a Factor XIIIa substrate domain.

15. The matrix of claim 14, wherein the second domain comprises SEQ ID NO:20.

16. A method for making a matrix comprising the steps of
a. providing at least one matrix capable of forming a crosslinked matrix,
wherein the matrix is selected from the group consisting of proteins, polysaccharides, glycoproteins, and synthetic materials.
b. adding an engineered protein or polysaccharide graft to the matrix,
wherein the engineered protein or polysaccharide graft comprises
a first domain comprising a bioactive factor or a polysaccharide,
a second domain, and
an enzymatic or hydrolytic degradation site between the first domain and the second domain, and
c. crosslinking the matrix, such that the matrix is linked to the second domain of the engineered protein or polysaccharide graft.

17. The method of claim 16, wherein the bioactive factor is selected from the group consisting of peptides, growth factors and enzymes.

18. The method of claim 17, wherein the bioactive factor is a heparin-binding growth factor.

19. The method of claim 18, wherein the heparin-binding growth factor is selected from the group consisting of transforming growth factor-beta, bone morphogenic proteins, fibroblast growth factor, vascular epithelial growth factor, interleukin-8, neurotrophin-6, heparin-binding epidermal growth factor, hepatocyte growth factor, connective tissue growth factor, midkine, heparin-binding growth associated molecules and mixtures thereof.

20. The method of claim 17, wherein the bioactive factor is peptide comprising at least one heparin-binding domain.

21. The method of claim 16, wherein the polysaccharide is selected from the group consisting of heparin, heparan sulfate and chondroitin sulfate.

22. The method of any of the claims 16 to 21, wherein the second domain is a substrate domain for a crosslinking enzyme.

23. The method of claim 23, wherein the second domain comprises a transglutaminase substrate domain.

24. The method of claim 23, wherein the second domain comprises a Factor XIIIa substrate domain.

25. An engineered protein or polysaccharide graft, comprising a first domain comprising a growth factor or a polysaccharide, a second domain that comprises a crosslinkable region that is capable of cross-linking the engineered protein or polysaccharide graft to a matrix, and an enzymatic or hydrolytic degradation site between the first and the second domain.

26. The engineered protein or polysaccharide graft of claim 25, wherein the bio-active factor is a heparin-binding growth factor.

27. The engineered protein or polysaccharide graft of claim 25, wherein the growth factor is selected from the group consisting of transforming growth factor-beta, bone morphogenic proteins, fibroblast growth factor, vascular epithelial growth factor, interleukin-8, neurotrophin-6, heparin-binding epidermal growth factor, hepatocyte growth factor, connective tissue growth factor, midkine, heparin-binding growth associated molecules and mixtures thereof.

28. The engineered protein or polysaccharide graft of claim 25, wherein the polysaccharide is selected from the group consisting of heparin, heparan sulfate and chondroitin sulfate.

29. The engineered protein or polysaccharide graft of any of claims 25 to 28, wherein the degradation site is an enzymatic degradation site, which is cleaved by an enzyme selected from the group consisting of plasmin and matrix metalloproteinase.

30. The engineered protein or polysaccharide graft of any of the claims 25 to 29, wherein the second domain is a substrate domain for a crosslinking enzyme.

31. The engineered protein or polysaccharide graft of claim 30, wherein the second domain comprises a transglutaminase substrate domain.

32. The engineered protein or polysaccharide graft of claim 31, wherein the second domain comprises a Factor XIIIa substrate domain.

33. The matrix of any one of claims 1 to 15 for repair, regeneration, or remodeling of tissues and/or release of bioactive factors.

## Patentansprüche

1. Vernetzte Matrix, die geeignet ist zum Wachsen oder Einwachsen von Zellen, bei der mindestens eine Aufpfropfung eines manipulierten Proteins oder Polysaccharids kovalent oder ionisch an die Matrix gebunden ist,
wobei die Aufpfropfung des manipulierten Proteins oder Polysaccharids
eine erste Domäne, die einen bioaktiven Faktor oder ein Polysaccharid aufweist,
eine zweite Domäne, wobei die Aufpfropfung des manipulierten Proteins oder Polysaccharids durch die zweite Domäne an die Matrix gebunden ist, und
eine enzymatische oder hydrolytische Abbaustelle zwischen der ersten Domäne und der zweiten Domäne aufweist.

2. Matrix nach Anspruch 1, wobei der bioaktive Faktor ein Peptid ist.

3. Matrix nach Anspruch 2, wobei das Peptid mindestens eine Heparin-bindende Domäne aufweist.

4. Matrix nach Anspruch 1, wobei der bioaktive Faktor ein Wachstumsfaktor oder ein Protein ist.

5. Matrix nach Anspruch 4, wobei der bioaktive Faktor ein Heparin-bindender Wachstumsfaktor ist.

6. Matrix nach Anspruch 5, wobei der Heparin-bindende Wachstumsfaktor ausgewählt ist aus der Gruppe, die aus transformierendem Wachstumsfaktor beta, knochenmorphogenetischen Proteinen, Fibroblasten-Wachstumsfaktor, Gefäßepithel-Wachstumsfaktor, Interleukin-8, Neurotrophin-6, Heparin-bindendem epidermalen Wachstumsfaktor, Hepatozyten-Wachstumsfaktor, Bindegewebe-Wachstumsfaktor, Midkine, Heparin-bindenden, mit Wachstum im Zusammenhang stehenden Molekülen und Gemischen davon besteht.

7. Matrix nach Anspruch 1, wobei der bioaktive Faktor ein Enzym ist.

8. Matrix nach Anspruch 1, wobei das Polysaccharid ausgewählt ist aus der Gruppe, die aus Heparin, Heparansulfat und Chondroitinsulfat besteht.

9. Matrix nach einem der Ansprüche 1 bis 8, wobei die Matrix ausgewählt ist aus der Gruppe, die aus Proteinen, Polysaccharid, Glycoproteinen und synthetischen Materialien besteht.

10. Matrix nach Anspruch 9, wobei die Matrix Fibrin aufweist.

11. Matrix nach Anspruch 1, wobei die Abbaustelle eine enzymatische Abbaustelle ist, die von einem Enzym gespalten wird, das ausgewählt ist aus der Gruppe, die aus Plasmin und Matrix-Metalloproteinase besteht.

12. Matrix nach einem der Ansprüche 1 bis 11, wobei die zweite Domäne eine Substratdomäne für ein vernetzendes Enzym ist.

13. Matrix nach Anspruch 12, wobei die zweite Domäne eine Transglutaminase-Substratdomäne aufweist.

14. Matrix nach Anspruch 13, wobei die zweite Domäne eine Faktor Xllla-Substratdomäne ist.

15. Matrix nach Anspruch 14, wobei die zweite Domäne SEQ ID NO:20 aufweist.

16. Verfahren zur Herstellung eines Matrix, folgende Schritte aufweisend:
a. Bereitstellen mindestens einer Matrix, die zur Bildung einer vernetzten Matrix in der Lage ist, wobei die Matrix ausgewählt wird aus der Gruppe, die aus Proteinen, Polysacchariden, Glycoproteinen und synthetischen Materialien besteht,
b. Zugeben einer Aufpfropfung eines manipulierten Proteins oder Polysaccharids zu der Matrix, wobei die Aufpfropfung des manipulierten Proteins oder Polysaccharids
eine erste Domäne, die einen bioaktiven Faktor oder ein Polysaccharid aufweist,
eine zweite Domäne, und
eine enzymatische oder hydrolytische Abbaustelle zwischen der ersten Domäne und der zweiten Domäne aufweist, und
c. Vernetzen der Matrix, so dass die Matrix an die zweite Domäne der Aufpfropfung des manipulierten Proteins oder Polysaccharids gebunden wird.

17. Verfahren nach Anspruch 16, wobei der bioaktive Faktor ausgewählt wird aus der Gruppe, die aus Peptiden, Wachstumsfaktoren und Enzymen besteht.

18. Verfahren nach Anspruch 17, wobei der bioaktive Faktor ein Heparin-bindender Wachstumsfaktor ist.

19. Verfahren nach Anspruch 18, wobei der Heparin-bindende Wachstumsfaktor ausgewählt wird aus der Gruppe, die aus transformierendem Wachstumsfaktor beta, knochenmorphogenetischen Proteinen, Fibroblasten-Wachstumsfaktor, Gefäßepithel-Wachstumsfaktor, Interleukin-8, Neurotrophin-6, Heparin-bindendem epidermalen Wachstumsfaktor, Hepatozyten-Wachstumsfaktor, Bindegewebe-Wachstumsfaktor, Midkine, Heparin-bindenden, mit Wachstum im Zusammenhang stehenden Molekülen und Gemischen davon besteht.

20. Verfahren nach Anspruch 17, wobei der bioaktive Faktor ein Peptid ist, das mindestens eine Heparin-bindende Domäne aufweist.

21. Verfahren nach Anspruch 16, wobei das Polysaccharid ausgewählt wird aus der Gruppe, die aus Heparin, Heparansulfat und Chondroitinsulfat besteht.

22. Verfahren nach einem der Ansprüche 16 bis 21, wobei die zweite Domäne eine Substratdomäne für ein vernetzendes Enzym ist.

23. Verfahren nach Anspruch 22, wobei die zweite Domäne eine Transglutaminase-Substratdomäne aufweist.

24. Verfahren nach Anspruch 23, wobei die zweite Domäne eine Faktor Xllla-Substratdomäne aufweist.

25. Aufpfropfung eines manipulierten Proteins oder Polysaccharids, aufweisend eine erste Domäne, die einen Wachstumsfaktor oder ein Polysaccharid aufweist, eine zweite Domäne, die einen vernetzbaren Bereich aufweist, der in der Lage ist, die Aufpfropfung des manipulierten Proteins oder Polysaccharids mit einer Matrix zu vernetzen, und eine enzymatische oder hydrolytische Abbaustelle zwischen der ersten und der zweiten Domäne.

26. Aufpfropfung eines manipulierten Proteins oder Polysaccharids nach Anspruch 25, wobei der bioaktive Faktor ein Heparin-bindender Wachstumsfaktor ist.

27. Aufpfropfung eines manipulierten Proteins oder Polysaccharids nach Anspruch 25, wobei der Wachstumsfaktor ausgewählt ist aus der Gruppe, die aus transformierendem Wachstumsfaktor beta, knochenmorphogenetischen Proteinen, Fibroblasten-Wachstumsfaktor, Gefäßepithel-Wachstumsfaktor, Interleukin-8, Neurotrophin-6, Heparin-bindendem epidermalen Wachstumsfaktor, Hepatozyten-Wachstumsfaktor, Bindegewebe-Wachstumsfaktor, Midkine, Heparin-bindenden, mit Wachstum im Zusammenhang stehenden Molekülen und Gemischen davon besteht.

28. Aufpfropfung eines manipulierten Proteins oder Polysaccharids nach Anspruch 25, wobei das Polysaccharid ausgewählt ist aus der Gruppe, die aus Heparin, Heparansulfat und Chondroitinsulfat besteht.

29. Aufpfropfung eines manipulierten Proteins oder Polysaccharids nach einem der Ansprüche 25 bis 28, wobei die Abbaustelle eine enzymatische Abbaustelle ist, die von einem Enzym gespalten wird, das ausgewählt ist aus der Gruppe, die aus Plasmin und Matrix-Metalloproteinase besteht.

30. Aufpfropfung eines manipulierten Proteins oder Polysaccharids nach einem der Ansprüche 25 bis 29, wobei die zweite Domäne eine Substratdomäne für ein vernetzendes Enzym ist.

31. Aufpfropfung eines manipulierten Proteins oder Polysaccharids nach Anspruch 30, wobei die zweite Domäne eine Transglutaminase-Substratdomäne aufweist.

32. Aufpfropfung eines manipulierten Proteins oder Polysaccharids nach Anspruch 31, wobei die zweite Domäne eine Faktor Xllla-Substratdomäne aufweist.

33. Matrix nach einem der Ansprüche 1 bis 15 zur Wiederherstellung, Regeneration oder Umgestaltung von Geweben und/oder zur Freisetzung von bioaktiven Faktoren.

## Revendications

1. Matrice réticulée convenant pour la croissance cellulaire ou la croissance cellulaire vers l'intérieur où au moins une greffe protéique ou polysaccharidique modifiée par ingénierie est liée de manière covalente ou ionique à la matrice,
où la greffe protéique ou polysaccharidique modifiée par ingénierie comprend
un premier domaine comprenant un facteur bioactif ou un polysaccharide,
un second domaine, où la greffe protéique ou polysaccharidique modifiée par ingénierie est liée à la matrice par le second domaine, et
un site de dégradation enzymatique ou hydrolytique entre le premier domaine et le second domaine.

2. Matrice selon la revendication 1 où le facteur bioactif est un peptide.

3. Matrice selon la revendication 2 où le peptide comprend au moins un domaine liant l'héparine.

4. Matrice selon la revendication 1 où le facteur bioactif est un facteur de croissance ou une protéine.

5. Matrice selon la revendication 4 où le facteur bioactif est un facteur de croissance liant l'héparine.

6. Matrice selon la revendication 5 où le facteur de croissance liant l'héparine est choisi dans le groupe consistant en le facteur de croissance transformant bêta, les protéines morphogènes osseuses, le facteur de croissance des fibroblastes, le facteur de croissance épithélial vasculaire, l'interleukine-8, la neurotrophine-6, le facteur de croissance épidermique liant l'héparine, le facteur de croissance des hépatocytes, le facteur de croissance de tissu conjonctif, la midkine, les molécules associées à la croissance liant l'héparine et leurs mélanges.

7. Matrice selon la revendication 1 où le facteur bioactif est une enzyme.

8. Matrice selon la revendication 1 où le polysaccharide est choisi dans le groupe consistant en l'héparine, l'héparine-sulfate et le chondroïtine-sulfate.

9. Matrice selon l'une quelconque des revendications 1 à 8 où la matrice est choisie dans le groupe consistant en les protéines, les polysaccharides, les glycoprotéines et les matières synthétiques.

10. Matrice selon la revendication 9 où la matrice comprend de la fibrine.

11. Matrice selon la revendication 1 où le site de dégradation est un site de dégradation enzymatique qui est clivé par une enzyme choisie dans le groupe consistant en la plasmine et une métalloprotéinase de matrice.

12. Matrice selon l'une quelconque des revendications 1 à 11 où le second domaine est un domaine de substrat pour l'enzyme réticulante.

13. Matrice selon la revendication 12 où le second domaine comprend un domaine de substrat de transglutaminase.

14. Matrice selon la revendication 13 où le second domaine est un domaine de substrat de facteur XIIIa.

15. Matrice selon la revendication 14 où le second domaine comprend SEQ ID NO:20.

16. Procédé pour produire une matrice comprenant les étapes de
a. fournir au moins une matrice capable de former une matrice réticulée, où la matrice est choisie dans le groupe consistant en les protéines, les polysaccharides, les glycoprotéines et les matières synthétiques,
b. ajouter une greffe protéique ou polysaccharidique modifiée par ingénierie à la matrice où la greffe protéique ou polysaccharidique modifiée par ingénierie comprend
un premier domaine comprenant un facteur bioactif ou un polysaccharide,
un second domaine, et
un site de dégradation enzymatique ou hydrolytique entre le premier domaine et le second domaine, et
c. réticuler la matrice de sorte que la matrice est liée au second domaine de la greffe protéique ou polysaccharidique modifiée par ingénierie.

17. Procédé selon la revendication 16 où le facteur bioactif est choisi dans le groupe consistant en les peptides, les facteurs de croissance et les enzymes.

18. Procédé selon la revendication 17 où le facteur bioactif est un facteur de croissance liant l'héparine.

19. Procédé selon la revendication 18 où le facteur de croissance liant l'héparine est choisi dans le groupe consistant en le facteur de croissance transformant bêta, les protéines morphogènes osseuses, le facteur de croissance des fibroblastes, le facteur de croissance épithélial vasculaire, l'interleukine-8, la neurotrophine-6, le facteur de croissance épidermique liant l'héparine, le facteur de croissance des hépatocytes, le facteur de croissance de tissu conjonctif, la midkine, les molécules associées à la croissance liant l'héparine et leurs mélanges.

20. Procédé selon la revendication 17 où le facteur bioactif est un peptide comprenant au moins un domaine liant l'héparine.

21. Procédé selon la revendication 16 où le polysaccharide est choisi dans le groupe consistant en l'héparine, l'héparane-sulfate et le chondroïtine-sulfate.

22. Procédé selon l'une quelconque des revendications 16 à 21 où le second domaine est un domaine de substrat pour une enzyme réticulante.

23. Procédé selon la revendication 22 où le second domaine comprend un domaine de substrat de transglutaminase.

24. Procédé selon la revendication 23 où le second domaine comprend un domaine de substrat de facteur XIIIa.

25. Greffe protéique ou polysaccharidique modifiée par ingénierie comprenant un premier domaine comprenant un facteur de croissance ou un polysaccharide,
un second domaine qui comprend une région réticulable qui est capable de réticuler la greffe protéique ou polysaccharidique modifiée par ingénierie à une matrice, et un site de dégradation enzymatique ou hydrolytique entre le premier et le second domaine.

26. Greffe protéique ou polysaccharidique modifiée par ingénierie selon la revendication 25 où le facteur bioactif est un facteur de croissance liant l'héparine.

27. Greffe protéique ou polysaccharidique modifiée par ingénierie selon la revendication 25 où le facteur de croissance est choisi dans le groupe consistant en le facteur de croissance transformant bêta, les protéines morphogènes osseuses, le facteur de croissance des fibroblastes, le facteur de croissance épithélial vasculaire, l'interleukine-8, la neurotrophine-6, le facteur de croissance épidermique liant l'héparine, le facteur de croissance des hépatocytes, le facteur de croissance de tissu conjonctif, la midkine, les molécules associées à la croissance liant l'héparine et leurs mélanges.

28. Greffe protéique ou polysaccharidique modifiée par ingénierie selon la revendication 25 où le polysaccharide est choisi dans le groupe consistant en l'héparine, l'héparane-sulfate et le chondroïtine-sulfate.

29. Greffe protéique ou polysaccharidique modifiée par ingénierie selon l'une quelconque des revendications 25 à 28 où le site de dégradation est un site de dégradation enzymatique qui a été clivé par une enzyme choisie dans le groupe consistant en la plasmine et une métalloprotéinase de matrice.

30. Greffe protéique ou polysaccharidique modifiée par ingénierie selon l'une quelconque des revendications 25 à 29 où le second domaine est un domaine de substrat pour une enzyme réticulante.

31. Greffe protéique ou polysaccharidique modifiée par ingénierie selon la revendication 30 où le second domaine comprend un domaine de substrat de transglutaminase.

32. Greffe protéique ou polysaccharidique modifiée par ingénierie selon la revendication 31 où le second domaine comprend un domaine de substrat de facteur XIIIa.

33. Matrice selon l'une quelconque des revendications 1 à 15 pour la réparation, la régénération ou le remodelage de tissus et/ou la libération de facteurs bioactifs.
